Europäisches Patentamt

**European Patent Office**

**Office européen des brevets**

(19)

(11) Publication number: **0 178 863**
**A1**

(12)

# EUROPEAN PATENT APPLICATION

(21) Application number: **85307299.9**

(22) Date of filing: **11.10.85**

(51) Int. Cl.⁴: **C 12 N 15/00**
C 12 P 21/00, C 07 H 21/04
C 12 N 5/00, C 12 N 1/20
//C12R1:19

(30) Priority: **15.10.84 US 660999**

(43) Date of publication of application:
**23.04.86 Bulletin 86/17**

(84) Designated Contracting States:
**AT BE CH DE FR GB IT LI NL SE**

(71) Applicant: SCHERING CORPORATION
2000 Galloping Hill Road
Kenilworth, New Jersey 07033(US)

(72) Inventor: **Reim, Richard Lewis**
78 Florence Avenue
Belleville New Jersey 07109(US)

(72) Inventor: **Leibowitz, Paul Jason**
185 Prospect Avenue
Hackensack New Jersey 07601(US)

(72) Inventor: **Narula, Satwant Kaur**
26 Natalie Drive
West Caldwell New Jersey 07006(US)

(72) Inventor: **Ryan, Michael Joseph**
34 Northwood Drive High Crest Lake
West Milford New Jersey 07480(US)

(74) Representative: **Ritter, Stephen David et al,**
Mathys & Squire 10 Fleet Street
London EC4Y 1AY(GB)

(54) Novel expression systems utilizing bacteriophage T7 promoters and gene sequences.

(57) Expression systems which utilize Bacteriophage T7 RNA polymnerase and promoter regions are described.

These systems are utilized to produce large quantities of useful polypeptides.

EP 0 178 863 A1

Case 2352

NOVEL EXPRESSION SYSTEMS UTILIZING BACTERIOPHAGE
T7 PROMOTERS AND GENE SEQUENCES.

It is now possible through the techniques of
genetic engineering to cause a host cell to produce
"heterologous" polypeptides, i.e., polypeptides which are
not naturally produced by that species of cell.  A number
of mammalian polypeptides have been produced in E. coli
such as somatostatin, described by Itakura et al.,
Science 198: 1056 (1977); the component A and B chains of
human insulin, disclosed by Goeddel et al., Proc. Nat.
Acad. Sci. USA 76: 106 (1979); human growth hormone,
disclosed by Goeddel et al., Nature 281: 411 (1980);
human fibroblast interferon, disclosed by Goeddel et al.,
Nucleic Acids Res. 8: 4057 (1980); and human serum
albumin, as disclosed by Lawn et al., Nucleic Acids Res.
9: 6103 (1981).

In order to cause a host cell to produce or
express a heterologous polypeptide, a structural sequence
(also commonly called a coding sequence or gene) which
codes for a polypeptide is usually placed near a promoter
sequence which causes the gene to be read or transcribed
into messenger RNA (mRNA).  The transcription of the gene
into mRNA is instituted when an enzyme known as RNA
polymerase contacts and interacts with the promoter
sequence; it thereafter moves along the gene to cause the synthesis of an mRNA molecule specified by the gene. The mRNA is thereafter translated into a specific polypeptide by cellular constituents, for example, ribosomes, transfer RNAs, etc.

A number of promoter sequences have been used to promote the expression of foreign genes (i.e., genes not normally present) in E. coli, such as the lac promoter, Backman and Ptashne, Cell 13: 65 (1978); the trp promoter, Hallewell and Emtage, Gene 9: 27 (1980); and the phage lambda $P_L$ promoter, Bernard et al., Gene 5: 59 (1979).

A number of gene promoter/operator systems are inducible; i.e., their activity can be varied substantially by the presence or absence of a certain substance or condition. For example, the lac promoter system of E. coli has a relatively low level of activity in the absence of lactose; in this condition, it is said to be repressed. However, in the presence of an inducer such as lactose itself or isopropyl-β-D-thiogalactoside (IPTG) the lac promoter system becomes derepressed and causes a high level of transcription of the gene sequence adjoining the promoter; see, e.g., J. Miller and W. Reznikoff, The Operon, 2nd Edition, Cold Spring Harbor Labs, New York (1982). Other inducible promoter systems include the trp promoter (which has relatively low activity if an excess of tryptophan is present, and higher activity if a low concentration of tryptophan is present or if 3-beta-indolylacrylic acid is present; see Hallewell and Emtage, supra), and the phage lambda $P_L$ promoter (which has a relatively low level of activity at about 30°C and higher activity at 41°C in the presence of a temperature-sensitive mutant lambda repressor; see Bernard et al., supra).

The promoter regions present in the DNA of bacteriophage T7 are of particular interest since upon

-3-

infection of bacterial cells, e.g. E. coli, by this bacteriophage the infected cell will almost immediately begin to produce only T7 bacteriophage polypeptides to the virtual exclusion of the host cell's own requirements. This action is, in large, a consequence of a very strong interaction between the bacteriophage T7 promoters and the bacteriophage RNA polymerase (T7 RNA polymerase). In a related action, bacteriophage T7 DNA is known to code for a protein which inactivates E. coli RNA polymerase by binding to it, thereby reducing transcription of E. coli DNA. It is due to these two factors that the protein synthesizing machinery of E. coli is given over almost exclusively to the production of bacteriophage protein shortly after infection.

It would, therefore, be very useful if the methods utilized by bacteriophage T7 to exclusively direct synthesis of bacteriophage-required proteins could be harnessed to direct the synthesis of polypeptides which are desired for medical and diagnostic research, and for other purposes.

A suggestion of the use of bacteriophage T7 promoters to direct the transcription of a cloned gene in bacteria is contained in McAllister et al., J. Mol. Biol., 153: 527 (1981). This reference postulates that the T7 RNA polymerase which would be required to initiate the T7-promoter-directed transcription would have to be supplied to the cell by infection or from the cloned T7 polymerase gene. The reference is silent as to any specific method for achieving this result.

Supplying T7 RNA polymerase by infection would seem to be of limited use since it is produced only briefly during infection and does not accumulate to high levels. Secondly, T7 bacteriophage infection would be accompanied by natural competition of T7 RNA polymerase for the cloned gene promoter and for promoter regions

0178863

-4-

present in the bacteriophage DNA itself. Also, since the infected cells lyse within a short time and too little of the desired polypeptide would be produced before lysis, this method would not appear practical.

The cloning and expression of the gene for T7 RNA polymerase (T7 Gene 1) is reported in a later paper (P. Davanloo et al., Proc. Nat. Acad. Sci. USA, 81: 2035 [1984]). The authors caution, however, that the presence of any T7 promoters in a plasmid containing T7 Gene 1 in the same orientation as the T7 promoters would have to be scrupulously avoided. They theorize that T7 RNA polymerase, by transcribing completely around such a plasmid would be able to direct the synthesis of its own mRNA from the cloned fragment that contained both the promoter and the intact T7 Gene 1. Such a construction, they believe, would lead to an autocatalytic increase both in the level of T7 RNA polymerase and in the rate of transcription of the plasmid; a condition which they say would almost certainly be lethal to the cell.

The authors state their belief that potentially a single molecule of active T7 RNA polymerase would be sufficient to trigger this response, so such a construction, i.e., a plasmid containing both a T7 promoter region and the T7 RNA polymerase gene, would be stable only if there was absolutely no expression of the cloned T7 RNA polymerase gene. This situation, they conclude, may be difficult or impossible to achieve. These authors, therefore, utilized a bacterial promoter sequence to direct the transcription of the T7 Gene 1, but were very careful to ensure that the plasmid construct did not contain an intact T7 promoter region. This is difficult to achieve since the bacteriophage DNA has no conveniently located restriction sites that will permit the ready isolation of a fragment which contains all of the T7 Gene 1 coding sequence without any promoter region.

-5-

Finally, the authors state their belief that it is unlikely that a sequence containing both the T7 Gene 1 translatable coding sequence and a T7 promoter region could be cloned on the same plasmid. Their attempt to place a promoter for T7 RNA polymerase into a plasmid along with the T7 Gene 1 produced only arrangements in which the promoter directs transcription opposite to the direction needed to transcribe the polymerase gene.

Thus, although the potential benefits of placing a foreign gene under the control of a T7 phage promoter are understood, such a convenient non-lethal method for providing the T7 RNA polymerase to a system comprising a T7 promoter has not previously been developed. The present invention provides a solution to this problem.

A system such as this is capable of working within any host such as various bacterial and yeast strains and mammalian or plant cell-lines so long as the synthesis of the T7 RNA polymerase is triggered by a regulatable host-specified promoter.

The invention therefore provides a method for producing a heterologous polypeptide in an appropriate host comprising culturing, under suitable conditions that favor the expression of the heterologous polypeptide, host cells which contain a recombinant plasmid vector that comprises a compatible inducible promoter region which is followed by and is in the same transcriptional direction (i.e. orientation) as the bacteriophage T7 RNA polymerase gene; and further which contain a bacteriophage T7 promoter region which is followed by and is in the same transcriptional direction as a structural DNA sequence which specifies said heterologous polypeptide; wherein said T7 promoter region and structural DNA are on the above-mentioned recombinant plasmid vector or on a second recombinant plasmid vector.

One feature of this method for producing a
heterologous polypeptide in an appropriate host comprises
culturing, under suitable conditions that favor the
expression of the heterologous polypeptide, host cells
which contain one or more copies each of:

a) a first recombinant plasmid vector that
comprises a compatible inducible promoter
region which is followed by and is in the
same orientation (i.e., transcriptional
direction) as the bacteriophage T7 RNA
polymerase gene; and

b) a second recombinant plasmid vector that
comprises a bacteriophage T7 promoter region
which is followed by and is in the same
transcriptional direction as a structural
DNA sequence which specifies said
heterologous polypeptide.

A second feature of this method for producing a
heterologous polypeptide in an appropriate host comprises
culturing, under suitable conditions that favor the
expression of the heterologous polypeptide, host cells
which contain one or more copies of a recombinant plasmid
vector, said vector comprising:

a) a compatible inducible promoter region which
is followed by and is in the same
transcriptional direction as the
bacteriophage T7 RNA polymerase gene; and

b) a bacteriophage T7 promoter region which is
followed by and is in the same
transcriptional direction as a structural
DNA sequence coding for said heterologous
polypeptide.

In this second method, the bacteriophage T7
promoter region can be oriented in the recombinant
plasmid vector in the same direction as or in the opposite direction to that required to transcribe the bacteriophage T7 RNA polymerase gene. We have obtained good expression of the structural DNA sequence when the bacteriophage T7 promoter region is oriented in the same direction as or in the direction opposite to that required to transcribe the bacteriophage T7 RNA polymerase gene.

Moreover, we have obtained particularly good expression of the structural DNA sequence by using bacterial host cells, e.g. E. coli, comprising an inducible bacterial promoter system.

A further feature of the invention is a DNA fragment which comprises a bacteriophage T7 promoter region which is coupled to and is in the same transcriptional direction as a structural DNA sequence which specifies a desired polypeptide. Such a DNA fragment is preferably contained in a recombinant plasmid vector which is suitable for transformation into a host (e.g. bacterial) cell.

Another feature of the invention is a DNA fragment which comprises:

    a) an inducible (e.g. bacterial) promoter region which is coupled to and is in the same transcriptional direction as the bacteriophage T7 RNA polymerase gene; and

    b) a bacteriophage T7 promoter region which is followed by and is in the same transcriptional direction as a structural DNA sequence which specifies a desired polypeptide.

Such a DNA fragment is preferably contained in a recombinant plasmid vector which is suitable for transformation into a host, e.g. bacterial, cell. In particular, the bacteriophage T7 promoter region is preferably oriented in the recombinant plasmid vector in the opposite direction to that required to transcribe the bacteriophage T7 RNA polymerase gene.

Another feature of the invention is a transformed host cell, especially a bacterium, which comprises at least one copy of a recombinant plasmid vector, said vector comprising a bacteriophage T7 promoter region which is coupled to and is in the same transcriptional direction as a structural DNA sequence which specifies a desired polypeptide. Such a transformed host cell, e.g. bacterium, preferably comprises in addition at least one copy of a second recombinant plasmid vector, said vector comprising an inducible bacterial promoter region which is coupled to and is in the same transcriptional direction as the bacteriophage T7 RNA polymerase gene.

Yet another feature of the invention is a transformed host, e.g. bacterium, which comprises at least one copy of a recombinant plasmid vector, said vector comprising:

a) a compatible (e.g. bacterial) inducible promoter region which is followed by and is in the same transcriptional direction as the bacteriophage T7 RNA polymerase gene; and

b) a bacteriophage T7 promoter region which is followed by and is in the same transcriptional direction as a structural DNA sequence which specifies a desired polypeptide.

In such a transformed bacterium, the bacteriophage T7 promoter region present in the recombinant plasmid vector is preferably oriented in the direction opposite to that required to transcribe the bacteriophage T7 RNA polymerase gene which is also present in said vector.

-9-

The desired polypeptide may for example be a naturally occurring polypeptide (e.g. an interferon) or a functionally equivalent analog, derivative on fragment thereof.

A still further feature of the invention is a recombinant plasmid vector comprising a bacteriophage T7 promoter region and, directly, preferably immediately, downstream thereof, a polylinker sequence for insertion of a structural DNA sequence specifying a desired polypeptide.

Bacteriophage T7 DNA was extracted from wild type phage T7 which can be obtained from several sources including the American Type Culture Collection (stock no. 11303B7).  The method of isolating the purified bacteriophage DNA was essentially as described in T. Maniatis et al., Molecular Cloning, Cold Spring Harbor Laboratory, pp. 76-85 (1982).  The DNA so obtained was restricted with various restriction endonucleases to yield the desired T7 bacteriophage promoters and structural gene fragments (e.g., as described in Methods Section herein).  The complete bacteriophage T7 DNA sequence is known; Dunn, J.J., and Studier, F.W., J. Mol. Biol. 166: 497 (1983).  The nomenclature utilized herein is described in this publication.

The T7 promoters are recognized to vary in their strength and have been allocated to three classes on this basis.  The strongest of these are the Class III promoters, the class of promoters preferred for the purposes of the invention.  One of the Class III promoters that regulates the expression of gene 10 protein (T7 Ø10) has been utilized in the invention. Quite unexpectedly, the lethality predicted in Proc. Nat. Acad. Sci. USA, 81: 2035 (1984) for a system comprising both the bacteriophage T7 RNA polymerase gene and a bacteriophage T7 promoter region on the same plasmid was not encountered. In fact, owing to the convenience of restriction sites present in bacteriophage T7 DNA, the bacteriophage T7 RNA polymerase gene utilized in the invention always was accompanied by a trailing intact T7 promoter region. This promoter region (T7 Ø1.1A) is in addition to the T7 promoter region utilized to initiate transcription of the gene for the desired polypeptide. As discussed above, this construction was predicted (Proc. Natl. Acad. Sci. USA, 81: 2035 [1984]) to result in an autocatalytic increase both in the level of T7 RNA polymerase and in the rate of transcription of the plasmid and thereby lead to the death of the cell. Surprisingly this morbidity was not encountered in practice, and the results are thus directly opposite to the teachings of the art.

The following description details two aspects of the invention whereby the expression of a gene which codes for a desired polypeptide may be placed under the control of a bacteriophage T7 promoter region. These aspects are identified as the "dual plasmid system" and the "single plasmid system."

A system such as this is capable of working within any host such as various bacterial and yeast strains, and mammalian or plant cell-lines so long as the synthesis of the T7 RNA polymerase is under the control of a regulatable host-specified promoter and the nucleotide coding sequence for the heterologous protein is under the control of a T7 promoter sequence.

## DUAL PLASMID SYSTEM

This system can function on any two vectors that are compatible in a chosen host. These vectors should contain conveniently located restriction sites and appropriate drug selection markers as are usual in recombinant DNA research experiments. Vector compatibility, i.e., their ability to co-exist in the same host cell, depends on the process of replication and partitioning employed by each of the vectors. Plasmids that share at least one common step in either of these processes would compete for maintenance, thereby leading to plasmid incompatibility. For example, it is known that pBR322, a Col El derivative, is compatible with pACYC-184 [Chang, A. C. Y. and Cohen, S. N., J. Bact, 134: 1141 (1978).

As a first step towards the construction of the dual plasmid system the lac UV5 promoter was cloned into pBR322 such that a unique restriction site could now be used to place a desired gene directly under the control of the lac UV5 promoter. T7 Gene 1 was cloned adjacent to the lac UV5 promoter at this restriction site, thereby facilitating the synthesis of T7 RNA polymerase on induction of the lac UV5 promoter by IPTG. The lac UV5 promoter constitutes two mutations in the RNA polymerase interaction site, which make it independent of CAP-cAMP regulation. The mutations have an enhancing effect on the system of the RNA polymerase-gene and structural DNA sequence, thereby leading to increased transcription of the structural DNA sequence. This property makes it a much stronger promoter than the wild type lac P. In order to obtain a functionally viable system, the expression of T7 RNA polymerase gene had to be tightly controlled. Therefore, the plasmid was transformed and maintained in a host strain comprising a chromosomally located lac I$^q$ repressor. This strain produces about 10 times more lac repressor molecules than does the wild type lac I$^+$ strain. E. coli RB791 is a lac I$^q$ strain which is available upon request from Dr. Barbara Backman, E. coli Genetic Stock Center, Yale University School of Medicine, New Haven, Connecticut.

The $I^q$ background should be suitably functional both as a plasmid-borne characteristic (P. Davanloo et al., _supra_) and when chromosomally located. In practice, any desired E. coli strain can be made amenable to this system by introducing into it a functional $I^q$ gene on a plasmid. Theoretically, an $I^q$ background may not be mandatory in the case where this system is present in a single or low copy format. Such a system could be effectively controlled by manipulating both the promoter strength and the gene copy number, such that the repressor molecules provided by the lac $I^+$ background are sufficient to repress the T7 Gene 1 synthesis effectively until induced.

Control of T7 Gene 1 expression in E. coli is not limited to a repressed lac po system. Various regulatory elements may be used to regulate its expression given an appropriate E. coli host background. For example, the bacteriophage lambda $P_L$ system may be used in which the promoter could be controlled by a temperature-sensitive repressor (eg. CI 857). T7 Gene 1 expression, if regulated by a temperature-sensitive repressor, would then be induced by raising the temperature.

The fragment bearing the T7 phage promoter of choice (T7 Ø10) was isolated by a series of restriction enzyme reactions and cloned into a pBR322 vehicle so that a convenient restriction site was reformed in order to be able to insert a gene coding for any desired polypeptide. Once the gene for the desired protein had been inserted and characterized, the resulting DNA sequence of T7 promoter and desired gene was transferred to a plasmid vector compatible with a pBR322-based vector. These cells, when induced with IPTG, produced the desired protein.

-13-

## SINGLE PLASMID SYSTEM

The plasmid, designated pRRB20, containing the lac UV5 promoter and T7 Gene 1 (described above) was opened at a unique restriction site, blunt-ended, and the fragment containing the phage T7 promoter and desired gene (obtained from the pBR322-based clone described above) was then ligated into the desired site. This resulted in a single pBR322-based plasmid that carried all the necessary functional entities for the T7 expression system. These plasmids, when transformed into a lac-I$^q$-containing host, enabled the isolation of stable clones. These clones, upon induction with IPTG, were found to produce large quantities of the desired protein.

In this single plasmid system, we encountered plasmids which comprise the DNA sequence of T7 Ø10 and desired gene oriented either in the same transcriptional direction as the DNA sequence of lac UV5 promoter and T7 Gene 1, or in the opposite transcriptional direction. Strains harboring both plasmid types are viable and show utility.

## BRIEF DESCRIPTION OF THE FIGURES

Figure 1 shows the structure of pRR19 containing the cloned 1136 base pair T7 DNA fragment encoding the carboxy-terminal sequence.

Figure 2 shows the structure of pRR-1T7-39 which contains the entire coding sequence of the T7 RNA polymerase gene and the T7 1.1A promoter sequence.

Figure 3 shows schematically the steps taken to clone the lac promoter sequence. Two restriction enzyme sites in pBR322 were changed. The Eco RI site was filled in and then religated creating an XmnI site resulting in p804-1. Subsequently in p804-1, the NdeI site was filled in and an Eco RI linker sequence was ligated into the site creating p804-RI. The lac promoter sequence, which existed as an EcoRI/PvuII fragment, could then be cloned into p804-RI. The resultant plasmid, p804-RI lac is shown and has the lac promoter sequence oriented in the counter-clockwise direction.

Figure 4 shows the construction of a plasmid containing the T7 RNA polymerase gene under the control of the lac promoter operator sequence.

Figure 5 shows the stepwise procedure used to clone the 512 base pair fragment encoding the T7 Gene 2 sequence into pBR322.

Figure 6 depicts the structure of pRR-2T7-35. The cloned fragment was identified and the orientation deduced using the restriction enzyme site KpnI as a marker.

Figure 7 shows how pRR-2T7-35 was reduced in size by cleaving out the ClaI/KpnI fragment forming pRR-1B-2T7.

Figure 8 shows how the NdeI site of pRR-1B-2T7 was changed to a BamHI site.

Figure 9 schematically shows the procedure used to clone T7 Gene 2 in tandem with the T7 Gene 1. T7 Gene 1 was cloned again into the PvuII region of p804-RI lac, with the ligation forming pRR-1T7 lac 5. The only difference between pRR-1T7 lac 5 and p1T7L-3 (Fig. 5) is that pRR-1T7 lac 5 has an XmnI restriction site where p1T7L-3 has an Eco RI site. T7 Gene 2 was cloned into pRR-1T7 lac 5 as a PstI/BamHI fragment forming pRR-1T7G2 lac 7. This sequence of T7 Genes 1 and 2 can be isolated with restriction enzyme Eco RI.

Figure 10 shows the structure of pAC-1T7G2-78. It was constructed by cloning the T7 Genes 1 and 2 Eco RI restriction fragment from pRR-1T7G2 lac 7 into the Eco RI restriction site of pACYC 184.

Figure 11 shows the steps employed to clone the T7 Gene 10 promoter, recreating the NdeI restriction site. Starting with the T7 genomic DNA a series of restriction enzyme digestions were made to obtain the 340 base pair fragment containing the T7 Gene 10 promoter. This was cloned with a fragment derived from pBR322. The plasmid isolated, pRR-T7Ø10-18 can be opened at the NdeI site so that any gene can be cloned into a position allowing the T7 promoter to direct its expression in the presence of T7 RNA polymerase.

Figure 12 schematically depicts how the T7 Gene 10 promoter was cloned in front of the alpha-2 interferon gene. The plasmid construction shown, pRR-T7Ø10-IF, has the alpha-2 interferon gene in a position to allow its expression by T7 RNA polymerase under the control of the T7 Gene 10 promoter.

Figure 13 shows schematically how pRR-T7Ø10-IF was digested with RsaI and cloned into pACYC 184 as a blunt end fragment. pAC-Ø10IF-2 consists of a T7 promoter directing the expression of a heterologous gene in a pACYC184 vehicle which is compatible with pBR322 in the same cell.

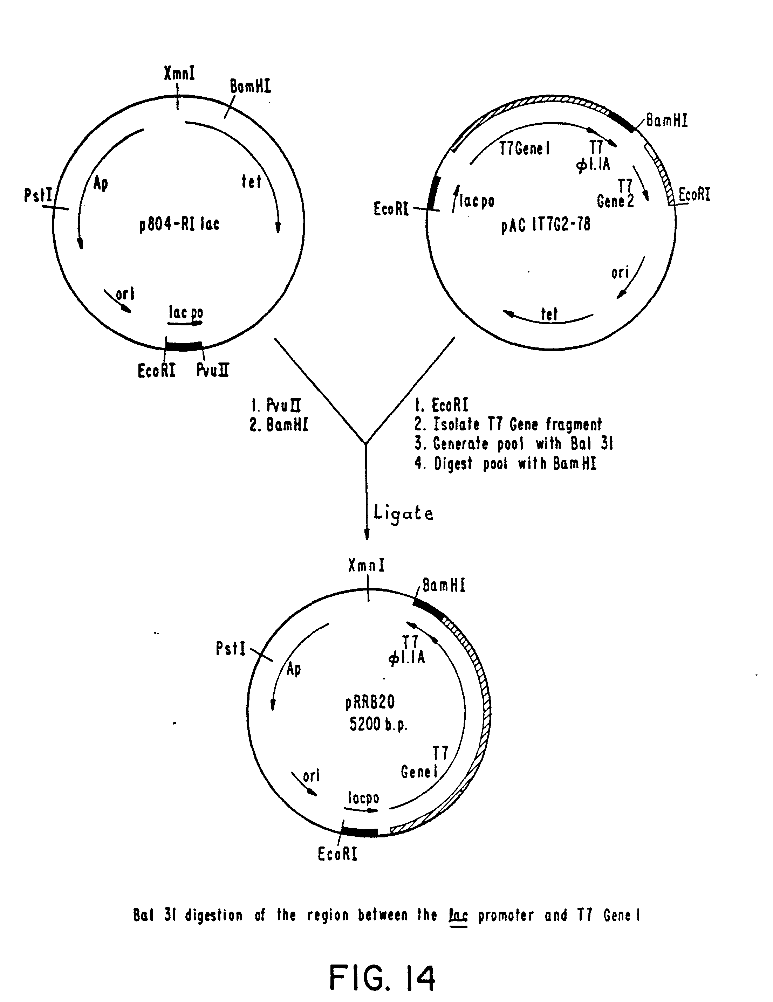

Figure 14 shows how a variety of clones were generated by digesting the T7 gene Eco RI fragment of pAC-1T7G2-78 with Bal 31. The enzyme was allowed to digest through the lac promoter region into the pBR322 DNA intervening between the promoter and the start of the T7 Gene 1 coding region. A pool of variable length fragments was then created by digestion with BamHI. These fragments were cloned adjacent to an intact lac promoter by insertion into a fragment obtained by digesting p804-RI lac with PvuII/BamHI. A variety of clones were isolated. One of them, pRRB20, was used in the two plasmid system and is shown in the Figure 14.

Figure 15 shows schematically the single plasmid system. A blunt end, RsaI fragment containing the T7 Gene 10 promoter - alpha 2 interferon complex was cloned into the Hind III site of pRRB20 which had been blunt ended. pRRB20IF-23 was isolated and the particular orientations of the genes and promoters involved in the T7 single plasmid system is illustrated.

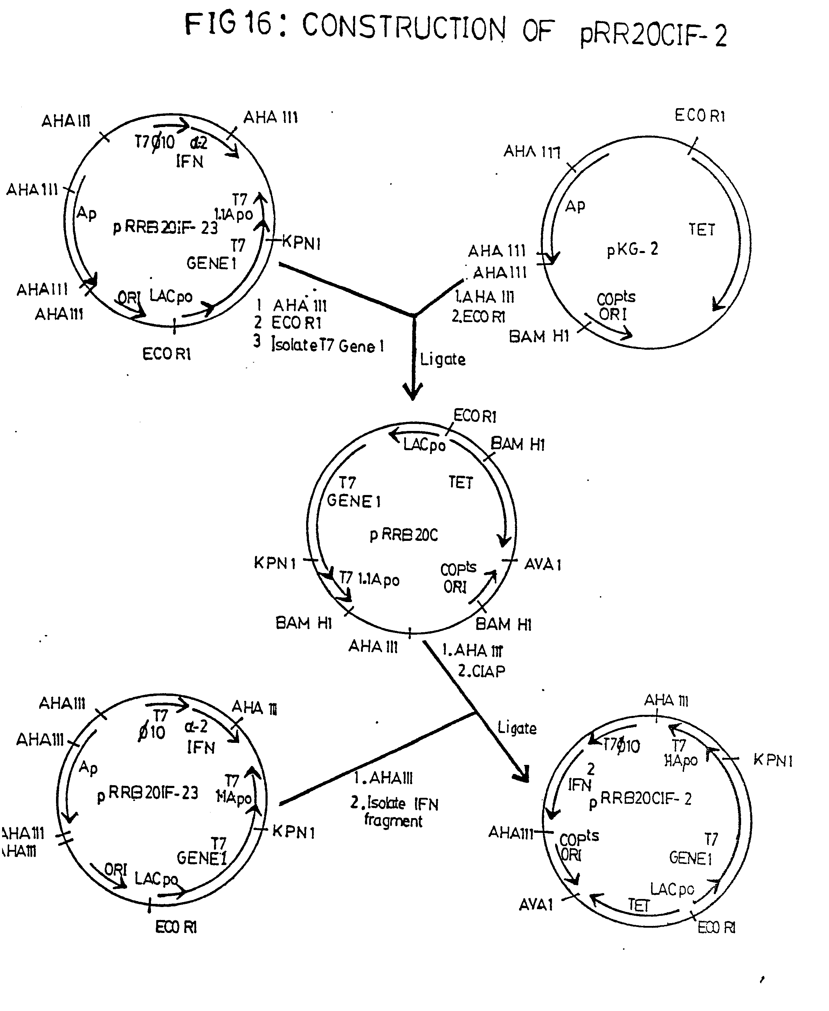

Figure 16 depicts the construction of the plasmid pRRB20C from pRRB20IF-23 and pKG-2. AhaIII/EcoRI restriction fragments were isolated from each plasmid. pRRB20IF-23 donated T7 Gene 1 to the construct and the fragment used from pKG-2 contained the tetracycline resistance gene and the $\underline{cop}^{ts}$ origin. This figure also shows how the T7$\emptyset$10-$\alpha$2IFN fragment from pRRB20IF-23 was cloned into the AhaIII restriction site. The fragment used was an AhaIII restriction fragment, and two AhaIII sites were recreated upon insertion of the DNA. pRRB20CIF-2 was an isolated construction in which the reading orientation of the T7 Gene 1 and T7 Gene 10 promoter-IFN complex were in the same 5' to 3' direction.

Figure 17 is a graph that depicts the effects of rifampicin on interferon production in E coli D1210/pRRB10IF-23. IPTG was added at a concentration of 2mM at zero time and the cells were incubated at 37$^{\circ}$C. Rifampicin (100 $\mu$g/ml) was added where indicated by the arrows (i.e. at 1.5, 5 and 6 hours).

Key to the graph is as follows: O: IFN level prior to rifampicin addition; X: IFN level in the culture when rifampicin was added at 1.5 hrs; ●: IFN level in culture when rifampicin was added at 5 hrs; ⊙: IFN levels in culture when rifampicin was added at 6 hours.

Figure 18 shows how pRRT7$\emptyset$10-Xu2 was constructed by cloning a fragment containing the T7 Gene 10 promoter into pMT11S. The promoter was positioned so that the unique restriction sites of the polylinker were 3' to the reading orientation of the promoter.

Figure 19 shows the construction of pRRTM$\emptyset$10-M$\alpha$2-1 by insertion of the murine alpha-2 IFN gene into the Sma I site of pRRT7∅10-Xu2. The murine alpha-2 IFN gene was cleaved from pTRG305-1 with Ava I and EcoRI restriction enzymes. The ends were blunt-ended by the Klenow reaction and the fragment was cloned into the blunt-ended SmaI restriction site of pRRT∅10-Xu2.

Figure 20 shows how the murine interleukin 3 (IL-3) was cloned into pRRT7∅10-Xu2. pTRP-Cll containing IL-3 was digested with ClaI enzyme and the site was filled in by the Klenow enzyme. The IL-3 gene was cleaved from the plasmid with Hind III and isolated by agarose gel electrophoresis. pRRT7∅10-Xu2 was prepared by digesting with SmaI and then Hind III. The IL-3 gene was then ligated into the vehicle and pRRT7∅10-IL-31 was isolated.

Figure 21 depicts the construction of pRRB20-MIF-215 and pRRB20IL-33. In each case a DNA fragment containing T7∅10 and murine gene was cleaved from the vector by digestion with ClaI and Hind III. pRRB20 was similarly digested with ClaI and Hind III and then treated with calf intestinal alkaline phosphatase; the T7∅10 and murine gene fragments were then ligated in, resulting in the cloned plasmids pRRB20-MIF-215 and pRRB20IL-33.

## MATERIALS AND METHODS

Bacterial Strains.

E. coli 294, a K12 derivative, was used as a host for all clonings except when T7 genes were placed under lac promoter control and in DNA sequence analyses experiments. The genotype of E. coli 294 (end A⁻, hsd R⁻, thi⁻, pro⁻) was described by Bolivar and Backman, 1979. E. coli D1210 (a derivative of E. coli HB101) was used as a host in cloning where T7 genes were placed under lac control. This strain was described by Sadler, et al., 1980, and contains chromosomally located lac I^q

and Y$^+$ genes. E. coli RB791 was obtained from the E. coli Genetic Stock Center, Yale University School of Medicine, New Haven, Connecticut. Its genotype is lac I p4000 (lac I$^q$), lac Z p4008 (lac L8), λ-, IN(rrn D - rrn E) 1, (R.B. Brent and M. Ptashne, 1981). E. coli (JM103) was used for cloning with M13 in order to sequence the intervening region between the lac promoter fragment and T7 Gene 1. This organism was described by Messing, 1979. Its genotype is Δ (lac, pro), thi$^-$, str A, end A, sbc B15, hsd R$^-$ sup E,F' tra D36 pro A$^+$, pro B$^+$, lac I$^q$, lac Z M15.

## Growth Medium.

A 20-10-5 TYE broth and agar medium was used throughout (20g Bacto-Tryptone, 10g Bacto Yeast Extract, 5g NaCl per liter H$_2$O) and was supplemented with appropriate antibiotics needed for selection (e.g., ampicillin, 75µg/ml or tetracycline HCl, 20µg/ml). Both antibiotics were purchased from Sigma Corp.

## Preparation of T7 phage DNA.

Wild type T7 bacteriophage was obtained and the T7 DNA was isolated and purified essentially by the methods described by Maniatis et al., 1982, for bacteriophage lambda except that the T7 bacteriophage were spun at 30K rpm overnight in a 70.1 Ti rotor. The concentration of CsCl was 0.81g per 1 ml of phage suspension.

## Plasmid vectors and other genetic elements.

A number of cloning vehicles were used; pBR322 (Sutcliffe, 1979); pACYC 184 (Chang and Cohen, 1978); pMT11S; and pKG-2. pMT11S (provided by H. Huang and K. Moore, DNAX Research Institute, Palo Alto, California) is a derivative of pBR322 containing a 66 b.p polylinker beginning with base pair 1 of the Eco RI restriction site. The entire 2275 b.p. DNA sequence is known. pKG-2 was constructed by K. Gewain, Schering Corp. It is a hybrid plasmid derived from pBR322 and PVU208 (Hakkaart et al., 1981). PstI/AvaI fragments from each plasmid were ligated so that the pVU208 plasmid donated the 3' end of the ampicillin resistance gene and the $\underline{cop}^{ts}$ origin. The 5' portion of the ampicillin resistance gene and the entire tetracycline resistance gene were donated by the pBR322 fragment.

The $\underline{lac}$ promoter ($\underline{lac}$ po) was originally described by Backman et al., 1976, and was originally derived from pKB252. The amino- terminal portion of T7 Gene 1 was isolated from pT7-13 (Stahl and Zinn, 1981), which was a gift from S. Stahl. The murine alpha-2 interferon sequence used was that isolated and characterized by Shaw et al., 1983. The murine interleukin-3 gene used was the sequence isolated and characterized by Yokota et al., 1984.

Testing of RRB strains for production of alpha-2 interferon in the dual plasmid system.

$\underline{E.\ coli}$ D1210 recombinants that contained the human alpha-2 interferon coding nucleotide sequences under T7 promoter control were tested for human alpha-2 interferon synthesis in the following manner. The recombinant organisms were streaked on 20-10-5 agar plates containing 100µg/ml ampicillin and 20µg/ml tetracycline HCl and incubated overnight at 30°C. In the morning, a 5ml suspension of cells in 20-10-5 broth was made from a plate scraping. The cell suspension was immediately diluted into 50 ml of 20-10-5 containing 30µg/ml ampicillin and 20µg/ml tetracycline in a 250 ml Erlenmeyer flask. The flasks were shaken at 250 rpm in a New Brunswick microtherm water bath shaker at 37°C. The cell turbidity was monitored with a Klett-Sommerson colorimeter at wavelength 500 nm (green filter) and when the density reached approximately Klett 200 the zero time sample was taken. At this time, IPTG was added to a final concentration of 5mM. A parallel control experiment was performed in which IPTG was not added to the culture. Triplicate 1 ml samples were taken every hour from both flasks for interferon assay and cell density.

The single plasmid system was tested for alpha-2 interferon production as follows: E. coli D1210 and E. coli RB791 were separately transformed with the pRRB20IF-23 construction and were separately tested for alpha-2 interferon production upon induction with IPTG. Cells were grown overnight on 20-10-5 agar plates containing 100 μg/ml ampicillin. After about 18 hours of growth, a 5 ml suspension of cells made from a plate scraping was diluted into 500 ml of fresh 20-10-5 broth containing 75 μg/ml ampicillin and was shaken at 300 rpm, at 30°C on a New Brunswick G-52 rotary shaker until a Klett O.D. of 200 was reached. At this time, 50 ml aliquots were transferred to 250 ml Erlenmeyer flasks and placed at 37°C in a New Brunswick microtherm water bath shaker. Zero time samples were taken and 5mM IPTG was added to the appropriate flasks. The flasks were shaken at 250 rpm and triplicate 1 ml samples were taken every two hours up to six hours post induction, and then once again after continuous overnight incubation (about 20 hours post induction).

Interferon Assay procedure.
One ml of culture was pipetted to a 1.5 ml Eppendorf tube, and the sample was centrifuged for 1 minute in a Brinkman microfuge. The supernatant was poured off and the pelleted cells were resuspended in 0.2 ml of lysing buffer (50mM Hepes, 30mM NaCl, 1% SDS, 1% ß-

0178863

-21-

Mercaptoethanol, 5M Urea; pH 7.0). The suspension was heated for 1 minute at 90°C and then diluted with cold phosphate buffered saline (PBS) to the original volume. The samples were vortexed and centrifuged in a Brinkman microfuge for 1 minute. 0.1 ml aliquots were withdrawn for dilution and subsequent assay.

The alpha-2 interferon protein is characterized as having an antiviral activity of at least $1 \times 10^7$ units/mg as determined by the cytopathic effect/inhibition assay employing EMC virus and human foreskin cells (FS-71) performed essentially as described in a publication by Familletti et al., 1981, using as a standard the NIH/WHO natural leukocyte interferon standard 69/19.

Enzymes and enzyme reactions.

All enzymes and linkers, except for Exo VII, were obtained from New England Biolabs, Inc., Beverly, Massachusetts. Exo VII enzyme was obtained from Bethesda Research Laboratories, Inc., Rockville, Maryland. The reactions were carried out essentially according to the specifications in the manufacturers' catalogs.

Electrophoresis and DNA isolation techniques.

Agarose gel electrophoresis and polyacrylamide gel electrophoresis techniques were carried out essentially as described by Maniatis et al., 1982. Isolation and recovery of DNA fragments from agarose or polyacrylamide gels were performed generally as described by H.O. Smith, 1980.

Large scale and small scale (mini) preparation of plasmids

The preparation technique used throughout this work for the large scale isolation of plasmids was the alkaline-SDS lysis method essentially as described in Maniatis et al., 1982. Large numbers of clones were also screened essentially by the alkaline-SDS lysis method described in Maniatis et al., 1982, except that the cells employed were generally obtained from a 10 ml overnight culture.

Transformation procedure.
The method for transforming E. coli was performed essentially as described by Dagert and Ehrlich, 1979.

DNA Sequencing.
The sequence of the promoter region of pRRB20 was determined by the M13 cloning and DNA sequencing system of New England Biolabs, Beverly Ma., which uses the cloning vectors developed by Messing and Vieira, 1982, and the sequencing techniques described by Sanger et al. 1977.

T7 and pBR322 genome locations.
Locations given for restriction enzyme sites and for promoters and genes on the T7 genome were determined from the sequence published by Dunn and Studier, 1983. Locations in this report are defined by the base pair (b.p.) number according to the convention of the above authors. Locations for restriction enzyme sites and base pair numbers in pBR322 are defined by the sequence described by Maniatis et al., 1982.

Murine Interferon Assay.
The sample generation and assay procedures for murine leukocyte interferon were identical to that used for the human alpha-2 interferon assay, except that the cells employed in the inhibition assay were L929 murine fibroblast cells. The standard used was human alpha-1 interferon.

SDS-Polyacrylamide Gel Electrophoresis.
SDS-polyacrylamide gel electrophoresis was performed
according to Laemmli, 1970.  The running gels were 15%
acrylamide and the stacking gels 6.6%.  The samples were
electrophoresed at 20 mA constant current until all of
the samples entered the running gel, at which time the
current was increased to 35 mA.


RESULTS

Cloning T7 Gene 1 (T7 RNA polymerase).  The T7 RNA
polymerase gene begins at b.p. 3171 and ends at b.p. 5820
of the T7 genome.  The entire gene was assembled from two
fragments cloned separately.  The carboxy terminal end of
the gene was cloned directly from T7 genomic DNA, as
follows:  The T7 genome was digested with Mbo I, and an
8311 b.p. fragment was isolated by agarose gel
electrophoresis.  This fragment was subsequently digested
with Nci I. Nci I sites at b.p. 2660 and b.p. 7589
yielded a 4929 b.p. fragment which was isolated by
agarose gel electrophoresis.  This fragment was then
further digested with Hae III.  A 1136 b.p. fragment,
representing the T7 genome from b.p. 4744 to b.p. 5880
and encompassing the T7 RNA polymerase 1.1A promoter, was
isolated by agarose gel electrophoresis.


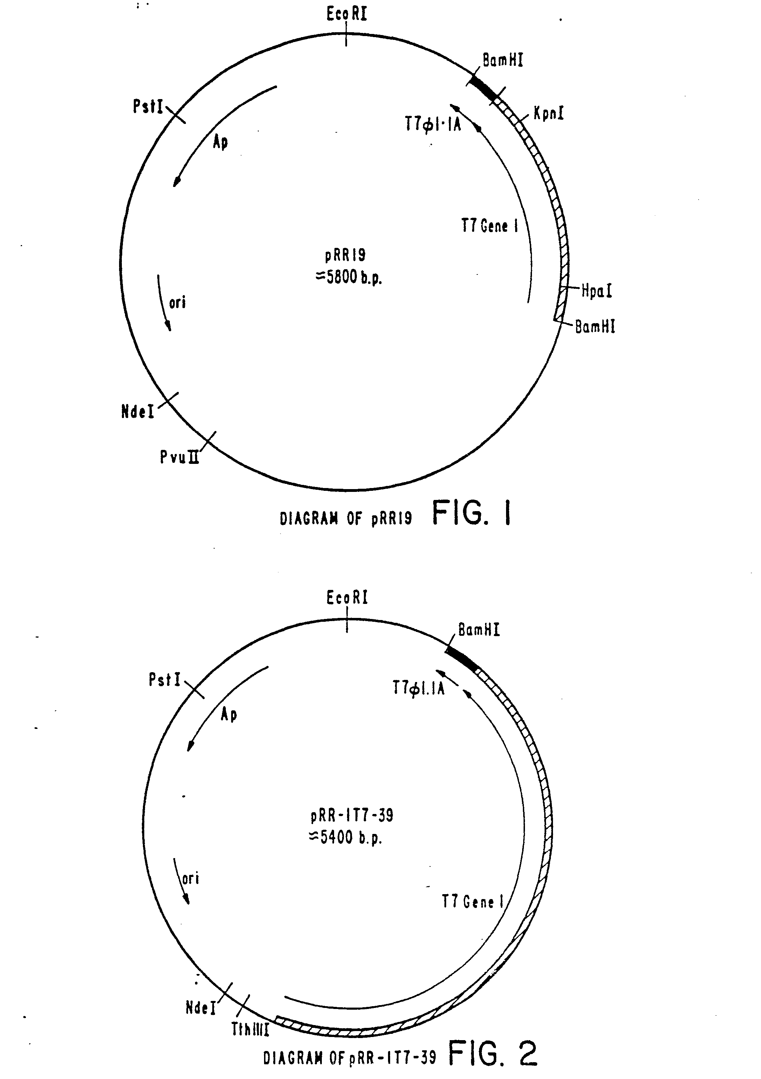

Construction of pRR19:  pBR322 was digested with Bam HI,
and the site was filled in with DNA polymerase I large
fragment (Klenow fragment).  The blunt-ended 1136 b.p. T7
fragment was then ligated into the Bam HI-
digested/filled-in/blunt-ended fragment derived from the
plasmid pBR322, and the ligated fragments were
transformed into E. coli 294.  The combination of the
filled-in Bam HI site and the Hae III site recreates a
Bam HI site, which was used as a marker to screen clones for the presence of the 1136 b.p. fragment. pRRl9 was isolated in this way (Figure 1). The orientation was deduced by observing the distance of the assymetrically placed Hpa I site present in the leading end of the 1136 b.p. fragment with respect to the nearby Hae III sites of the pBR322 plasmid.

Construction of pRR-1T7-39: The amino-terminal coding sequence of T7 Gene l was obtained from pT7-13 and was joined to its carboxy-terminal coding sequence contained in pRRl9 by taking advantage of the Kpn I site common to both fragments. This site is located in the T7 genome at b.p. 5614.

pRRl9 was digested with Kpn I and Pvu II. The large fragment containing the origin of replication and ampicillin resistance gene was isolated by agarose gel electrophoresis. pT7-13 was digested with Pst I, blunt-ended using the Klenow enzyme and then cut with Kpn I. The fragment containing T7 Gene l was isolated by agarose gel electrophoresis and ligated to the pRRl9 vehicle prepared above. The ligated fragments were transformed into E. coil 294. Clones were screened by digesting the plasmid DNA with Kpn I and Hpa I. The plasmid pRR-1T7-39 (Figure 2) was isolated from this screen. The T7 DNA contained in this plasmid extends from b.p. 3117 to b.p. 5880 of the T7 genome.

The lac promoter (lac po) system. The lac po sequence used was a UV5 derivative. The original promoter fragment existed as a 203 b.p. Hae III fragment and is referenced in reference 1. The sequence used in this construction consisted of a 125 b.p. fragment bracketed by Eco RI and Pvu II sites. It was constructed as follows: pKB252 was digested with Eco RI and the fragments were separated on a 10%-40% glycerol gradient which was run for 24 hours at 24K rpm in an SW 27 rotor. The small fragment was collected by pooling several gradients and was then digested with Alu I. pBR322 was digested with Eco RI and Pvu II and the large fragment containing the origin of replication and the ampicillin-resistance gene was isolated by agarose gel electrophoresis. The recovered pBR322 DNA was then ligated to the Alu I digested small DNA fragment and the ligated mixture was transformed into E. coli 294 and plated on 20-10-5 agar plates containing ampicillin and 5-bromo-4-chloro-3-indolyl-β-D-galactoside. Of the screened clones, one had a 125 b.p fragment. The nucleotide sequence of the cloned 125 b.p. fragment was determined to be the following:

GAATTCCAGTGAATC CGTAATCATG GTCATAGCTC ACTCATTAGG
CACCCCGGC TTTACACTTT ATGCTTCCGG CTCGTATAAT GTGTGGAATT
GTGAGCGGTA ACAATTTCAC ACAGGAAACAG

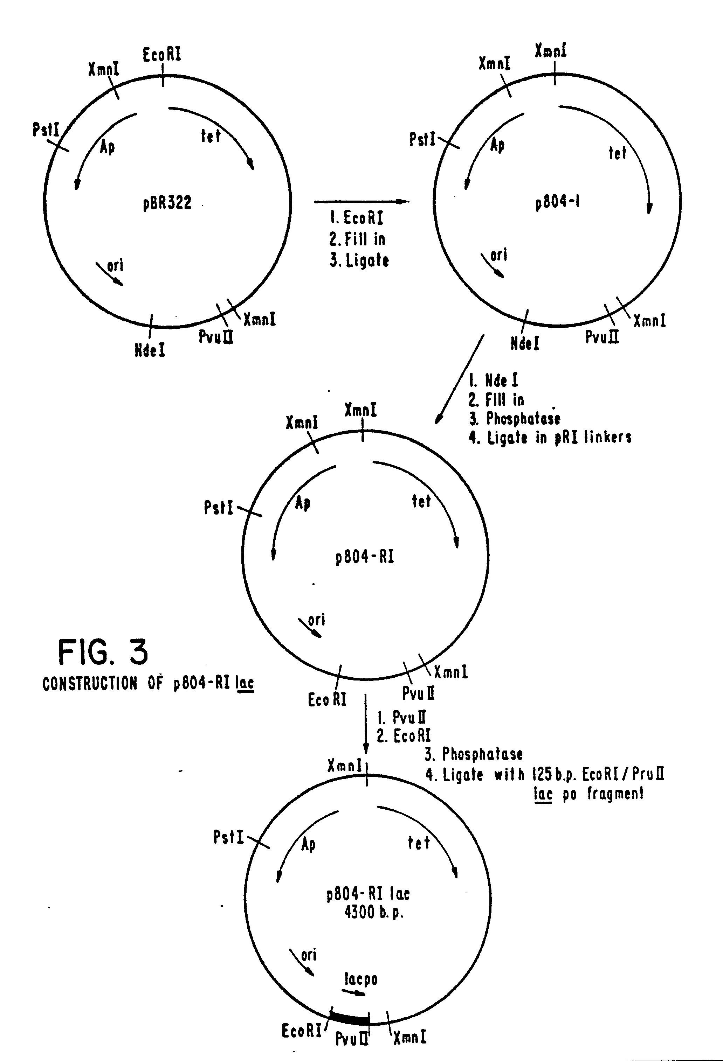

The 125 b.p. fragment was cloned into the Eco RI/Pvu II region of p804-RI which was constructed from pBR322 (Figure 3). In this scheme, the original pBR322 Eco RI site was filled in and religated (creating an Xmn I site), and Eco RI linkers were inserted into the filled-in Nde I site. After insertion of the 125 b.p. lac po fragment, the resultant plasmid was designated p804-RI lac (Figure 3 bottom).

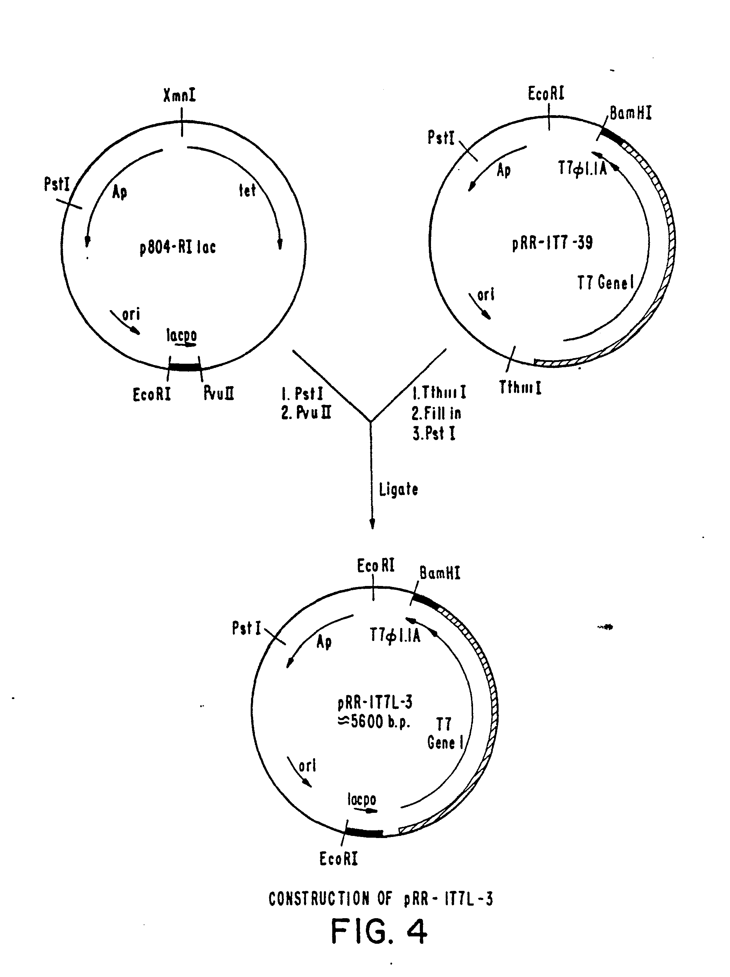

Cloning T7 Gene 1 under lac po control (refer to Figure 4). pRR-1T7-39 was digested with Tth111 I, the digested site was filled in, and the plasmid was then digested with Pst I. The T7 gene fragment was isolated by agarose gel electrophoresis. p804-RI lac was digested with Pvu II and Pst I. The fragment containing the lac po and the origin of replication was isolated by agarose gel electrophoresis and ligated to the isolated pRR-1T7-39 T7 Gene 1 fragment. The DNA was transformed into E. coli D1210. From the screened clones, pRR-1T7L-3 was isolated (Figure 4, bottom).

E. coli D1210 cells carrying pRR-1T7L-3 did not grow on 20-10-5 agar in the presence of isopropyl-β-D-thiogalactoside (IPTG), suggesting that T7 Gene 1 product was inhibitory to E. coli cells. This was tested in an experiment in which IPTG was added to broth cultures containing cells harboring T7 Gene 1 under lac po control. When cells growing in the log phase at 37° were induced with IPTG, there was a gradual cessation of growth followed by a death phase. The onset of the death phase is even more pronounced at 42°C. IPTG (2mM) containing agar plates were subsequently used to initially screen for any recombinant constructions in which T7 Gene 1 was under the lac po control.

Cloning of T7 Gene 2. T7 Gene 2 is located from b.p. 8898 to b.p. 9090 of the T7 genome. The protein's function is to inactivate the host RNA polymerase. The largest Hae III fragment of the T7 genome, which extends from b.p. 7579 to b.p. 10309 and encompasses T7 Gene 2, was isolated by agarose gel electrophoresis. The isolated DNA was then further digested with Hinc II. The Hinc II restriction sites at b.p. 8765 and b.p. 9277 bracket T7 Gene 2. This 512 b.p. fragment was isolated by agarose gel electrophoresis.

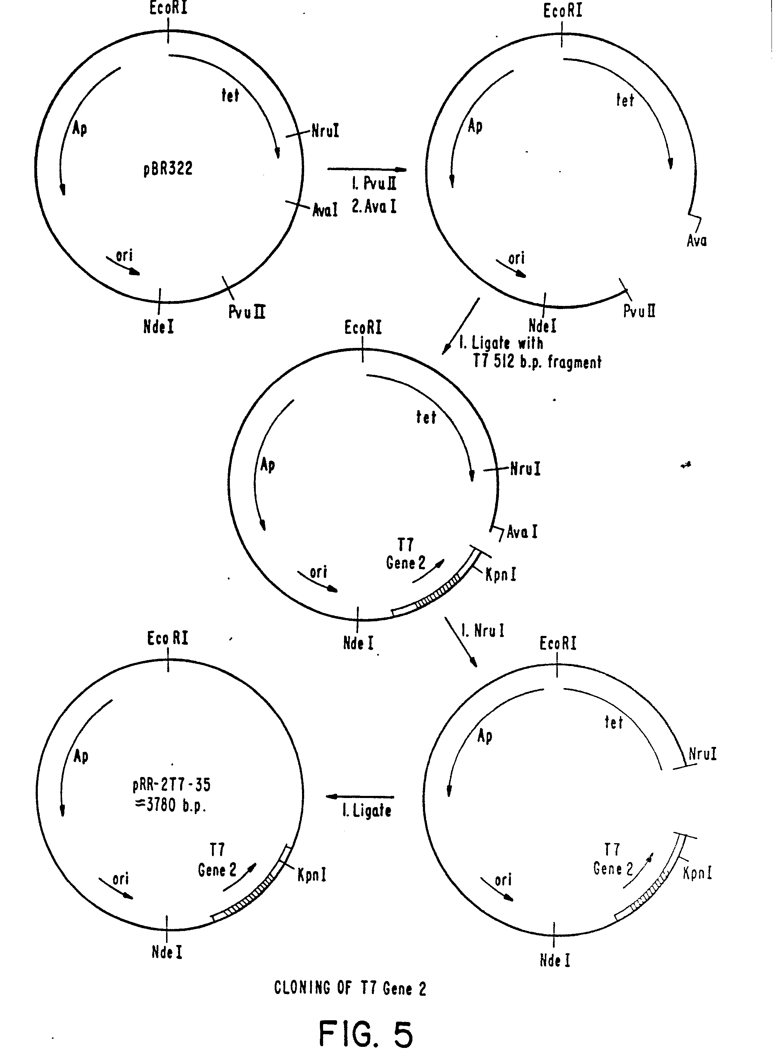

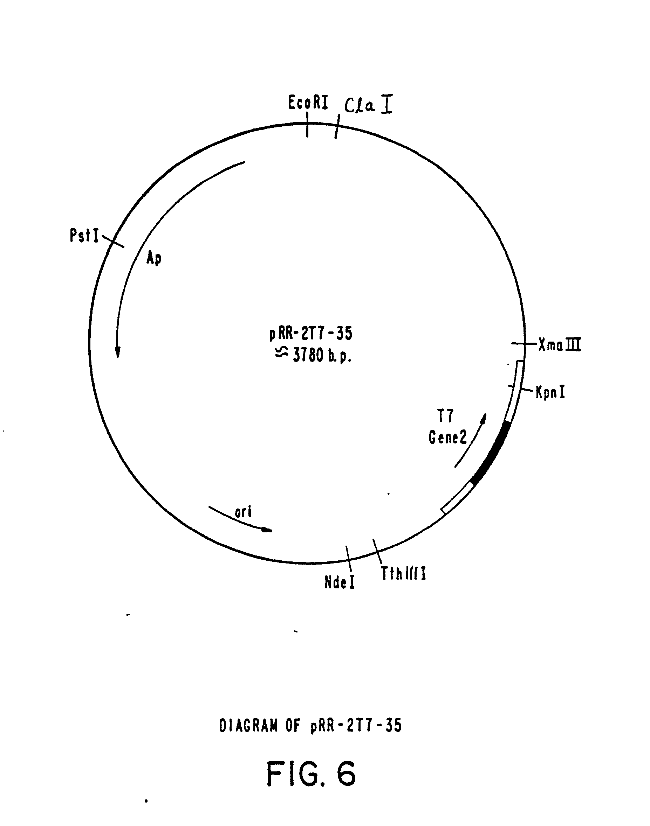

The cloning vehicle was prepared from pBR322. The plasmid was digested with Pvu II and Ava I. The large fragment containing the origin was isolated by agarose gel electrophoresis. The flush-ended 512 b.p. T7 fragment DNA was then ligated to the flush end (Pvu II site) of the pBR322 fragment; the Ava I end of this fragment remained free since it had a four-base single-strand overhang (Figure 5). The ligation was stopped by heating at 65°C for 10 min. and the DNA was recovered after precipitation with 2 volumes of ethanol. The pellet was redissolved in TE buffer and the solubilized DNA was then digested with Nru I (cleaving at a unique site in pBR322) which also leaves a flush end. The restricted DNA was run on an agarose gel. Bands in the appropriate linear size range were cut out and isolated. These linear DNA fragments were separately recircularized by ligating the blunt ends together and each was transformed into E. coli 294. Clones from each of the transformations were screened by cutting plasmid DNA with Kpn I, cleaving at a unique site within the 512 b.p. fragment region (b.p. 9190 of the T7 genome). One of these transformations yielded a clone which on further inspection proved to contain the 512 b.p. DNA fragment in the orientation shown in Figure 6. This plasmid was named pRR-2T7-35.

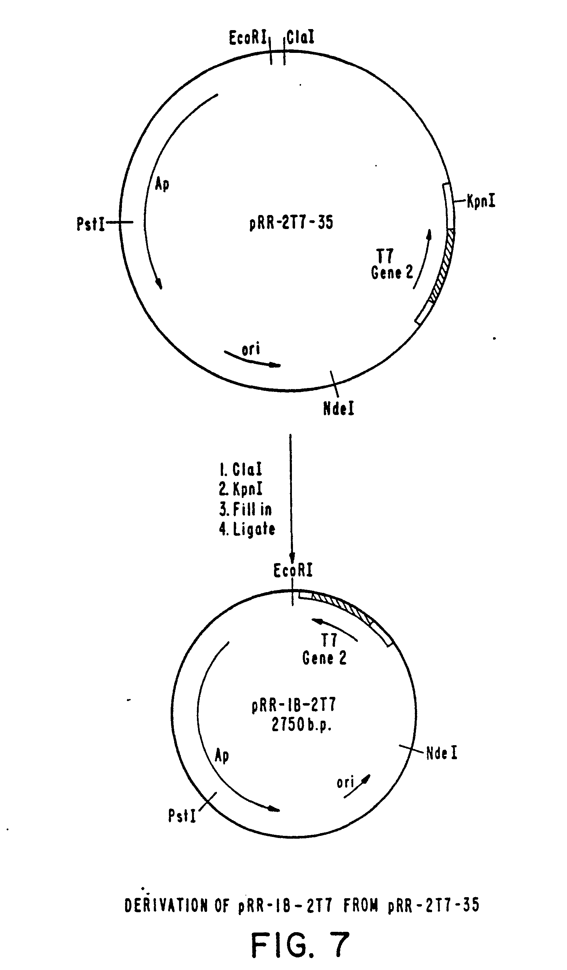

Unnecessary DNA was removed from this plasmid prior to combining gene 2 with T7 Gene 1. This unnecessary DNA was the pBR322 DNA between the Cla I site and the T7 Kpn I site, and it was removed as follows: pRR-2T7-35 was digested with Cla I and Kpn I, and the large fragment was isolated (Figure 7). This DNA fragment was treated with the Klenow enzyme. In this reaction, the Cla I site was filled in, and the Kpn I site, which has a 3' overhang, was blunt-ended. The two blunt ends were then ligated, and the plasmid DNA was transformed into E. coli 294. The clones were screened by digesting the DNA with the enzyme Hae III. From these clones, pRR-1B-2T7 was isolated (Figure 7).

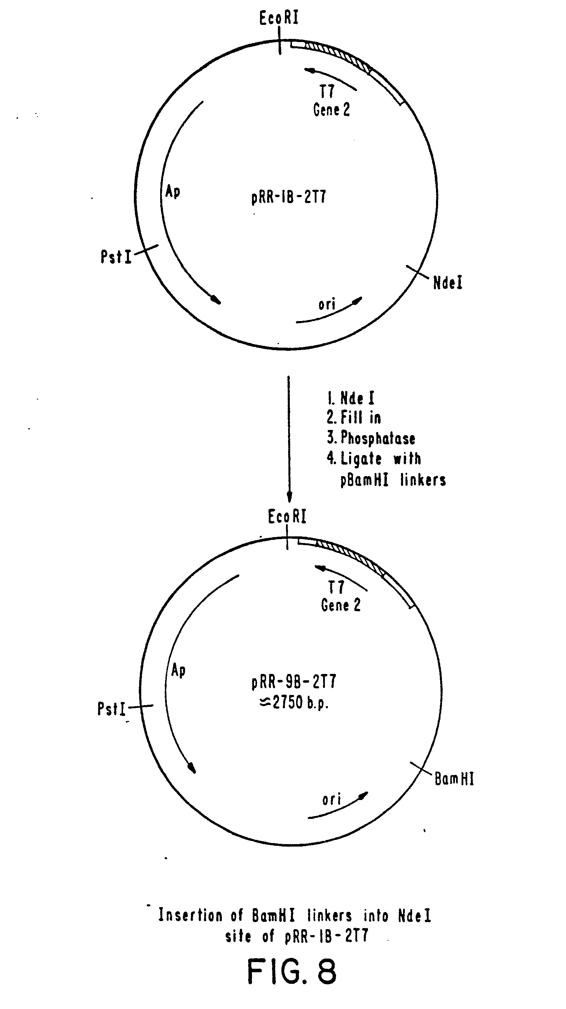

For the transfer of T7 Gene 2 to the T7 Gene 1 system, Bam HI linkers were inserted into the Nde I site of pRR-1B-2T7. This was done as follows: The plasmid was digested with Nde I, and the site was filled in with the Klenow fragment. The filled-in site was then treated with calf intestinal alkaline phosphatase (CIAP). 5'-phosphorylated Bam HI linkers were ligated into the blunt ends and the ligated DNA was transformed into E. coli 294. The clones were screened by digesting plasmid DNA with Bam HI. pRR-9B-2T7 was isolated and used in subsequent T7 Gene 2 experiments (Figure 8).

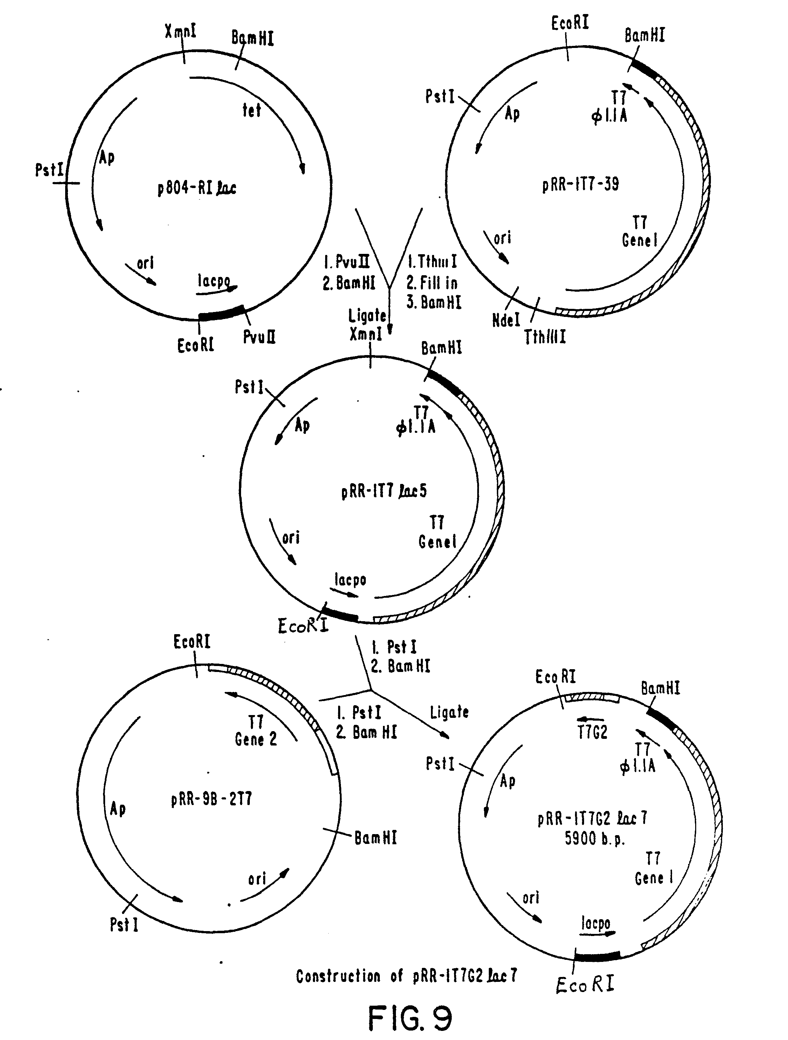

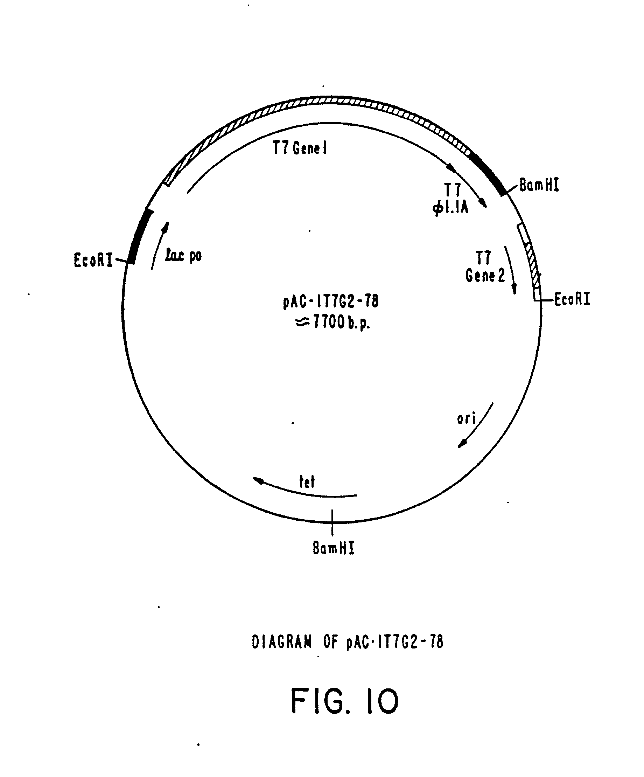

T7 Genes 1 and 2, and the lac promoter were combined and cloned into pACYC 184 by the following scheme (Figures 9 and 10). p804-RI lac was digested with Pvu II and Bam HI. The fragment containing lac po was isolated by agarose gel elecrophoresis. pRR-1T7-39 was digested with Tth111 I, and the site was filled in with the Klenow reaction. Bam HI was used to remove the fragment containing T7 Gene 1. After isolation by agarose gel electrophoresis and subsequent ligation to the above prepared p804-RI lac vehicle, the resultant plasmids were transformed into E. coli D1210. Clones were initially screened by looking for the absence of growth on IPTG containing agar plates relative to control plates. From these clones, pRR-1T7 lac 5 was isolated (Figure 9 center).

pRR-1T7 lac 5 and pRR-9B-2T7 were digested with Pst I and Bam HI. The fragments containing T7 Genes 1 and 2, respectively, were isolated by agarose gel electrophoresis, ligated together, and transformed into E. coli D1210. Clones were screened by digesting plasmid DNA with Eco RI. From this screen, pRR-1T7G2 lac 7 was

isolated (Figure 9 bottom).  The Eco RI fragment
containing <u>lac</u> po and T7 Genes 1 and 2 was digested out
of pRR-1T7G2 <u>lac</u> 7 and cloned into the Eco RI site of
pACYC 184; the resulting plasmids were transformed into
E. coli D1210, using the pACYC 184 tetracycline
resistance marker for selection.  One such plasmid was
designated pAC-1T7G2-78 (Figure 10).

<u>Cloning the T7 gene 10 promoter.</u>  The T7 Gene 10 promoter
is a strong promoter regulating the synthesis of at least
two genes; Genes 10A and 10B, both of which code for T7
bacteriophage head proteins.  This promoter is recognized
only by T7 RNA polymerase in the E. coli-T7 phage
system.  The promoter is located at b.p. 22,903 of the T7
genome.  This sequence is bracketed by two Nci I sites
(b.p. 21,839 and b.p. 23,192).

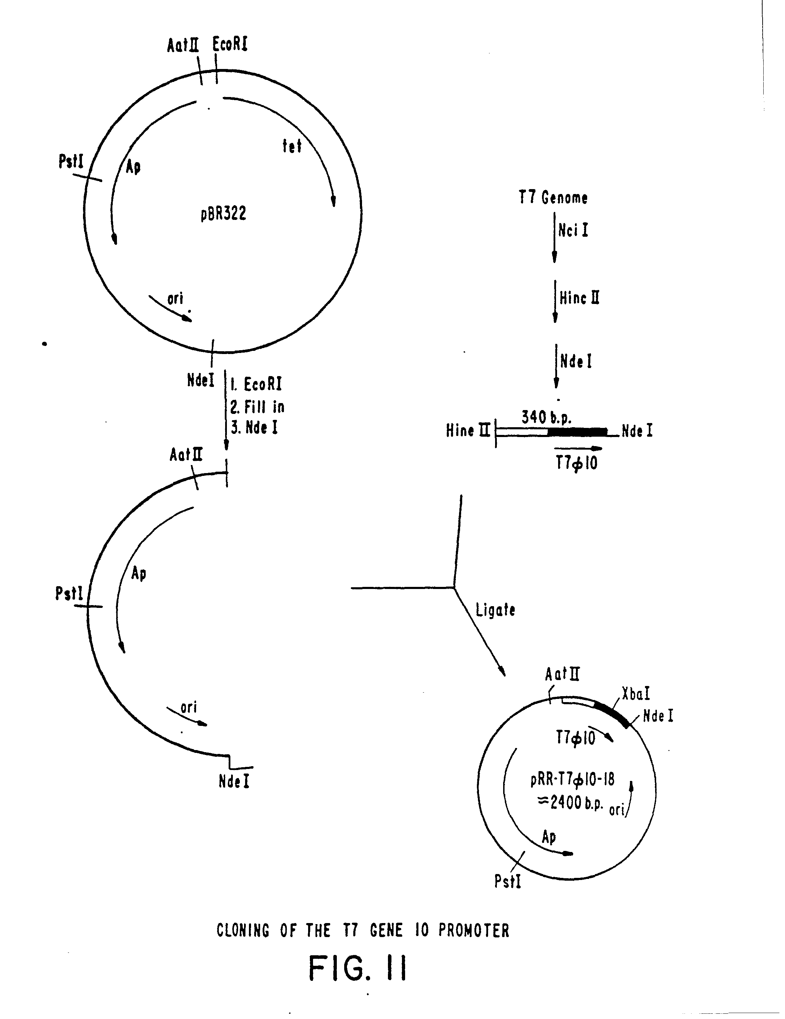

When Nci I enzyme was used to digest the T7 genome, a
1353 b.p. fragment was obtained containing the
promoter.  After isolation by agarose gel
electrophoresis, the fragment was further digested with
Hinc II and Nde I to give a 342 b.p. fragment resulting
from cuts at b.p. 22,622 and b.p. 22,964 of the T7
genome.  The Nde I site contains the ATG start codon of
gene 10A at b.p. 22,966.  This 342 b.p. fragment was
isolated by polyacrylamide gel electrophoresis and, after
purification, was cloned into a pBR322 vehicle digested
with Eco RI and Nde I.  The Eco RI site was filled in
with the Klenow fragment prior to the Nde I reaction
(Figure 11). The ligation mixture was transformed into E.
coli D1210.  Clones were initially screened by digesting
mini-preparations with Xba I since there is a unique Xba
I site in the T7 342 b.p. fragment between the promoter
and the ATG codon at b.p. 22,926.  From this screen, pRR-
TØ10-18 was isolated (Figure 11).

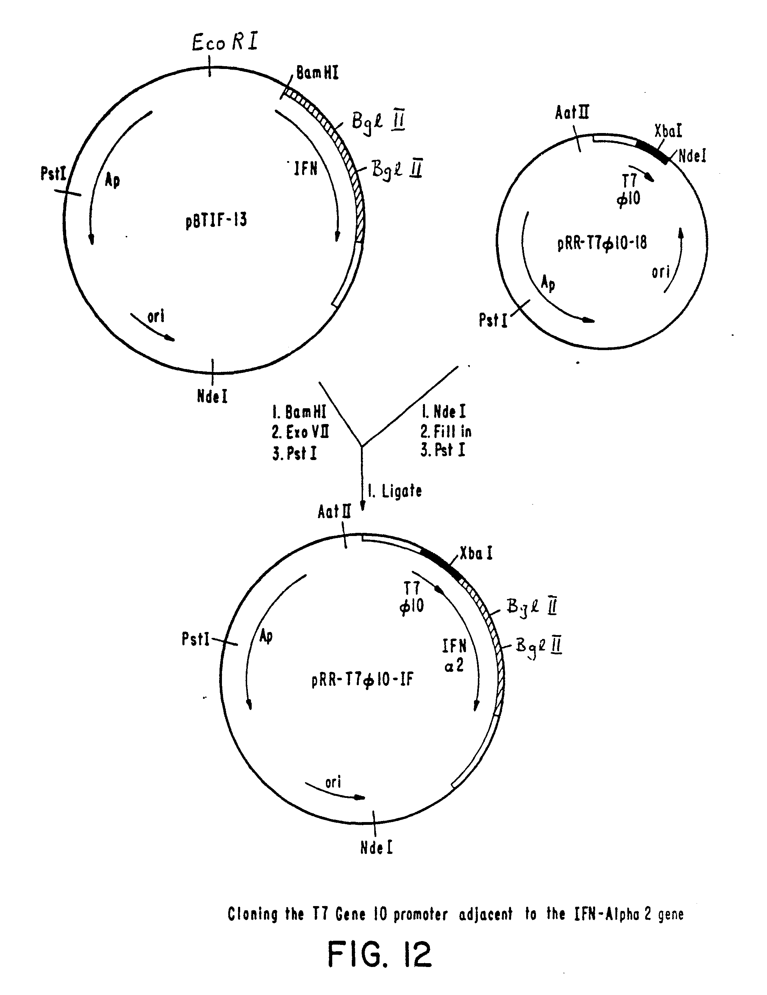

Connecting the alpha-2 interferon gene with the T7 Gene
10 promoter. To test the entire expression system, the
alpha-2 interferon gene (alpha-2 IFN) was chosen. The T7
Gene 10 promoter was cloned into pBTIF-13, which is a
pBR322 derivative with the alpha-2 IF gene cloned between
the Bam HI/Pvu II sites. It is under the control of the
tryptophan promoter which is located between the Eco RI
site and the Bam HI site in this plasmid. Figure 12
illustrates the cloning scheme and shows the fragments
assembled. pRR-T7Ø10-18 was digested with Nde I, the
site was filled in with the Klenow fragment, further
digestion was carried out with Pst I, and the promoter-
containing fragment was isolated by agarose gel
electrophoresis.

pBTIF-13 was digested with Bam HI, the site was blunt-
ended using Exo VII, further digestion was carried out
with Pst I, and the fragment containing the alpha-2 IF
gene isolated by agarose gel electrophoresis. The
promoter and IF gene fragments were ligated and
transformed into E. coli D1210. pRR-T7Ø10-IF was
isolated (Figure 12) by screening for a unique
Xba I/Bgl II fragment created by the juncture between the
T7 Gene 10 promoter and the alpha-2 IF gene.

Cloning the T7Ø10IF fragment to pACYC 184. The fragment
containing the T7 gene 10 promoter and alpha-2 IF gene
was cloned into pACYC-184 in the following scheme (Figure
13). This fragment is bracketed by two Rsa I sites present
in the pBR322 portion of pRR-T7Ø10IF (originally located
at b.p. 2282 and b.p. 3847 of pBR322). When digested
with Rsa I, pRR-T7Ø10-IF yielded a blunt-ended fragment
approximately 1880 b.p. in length. pACYC-184 was
digested with Eco RI, the site was filled in by the
Klenow reaction, and the ends were then treated with calf intestinal alkaline phosphatase; the product was then ligated with the Rsa I fragment isolated above and transformed into E. coli D1210. The clones were screened by restriction analysis with Xba I enzyme. pACYC-184 has a single Xba I restriction site, and since one exists in the T7 promoter region of the Rsa I fragment, two bands were observed using this screen. pAC-∅10IF-2 was isolated along with other clones.

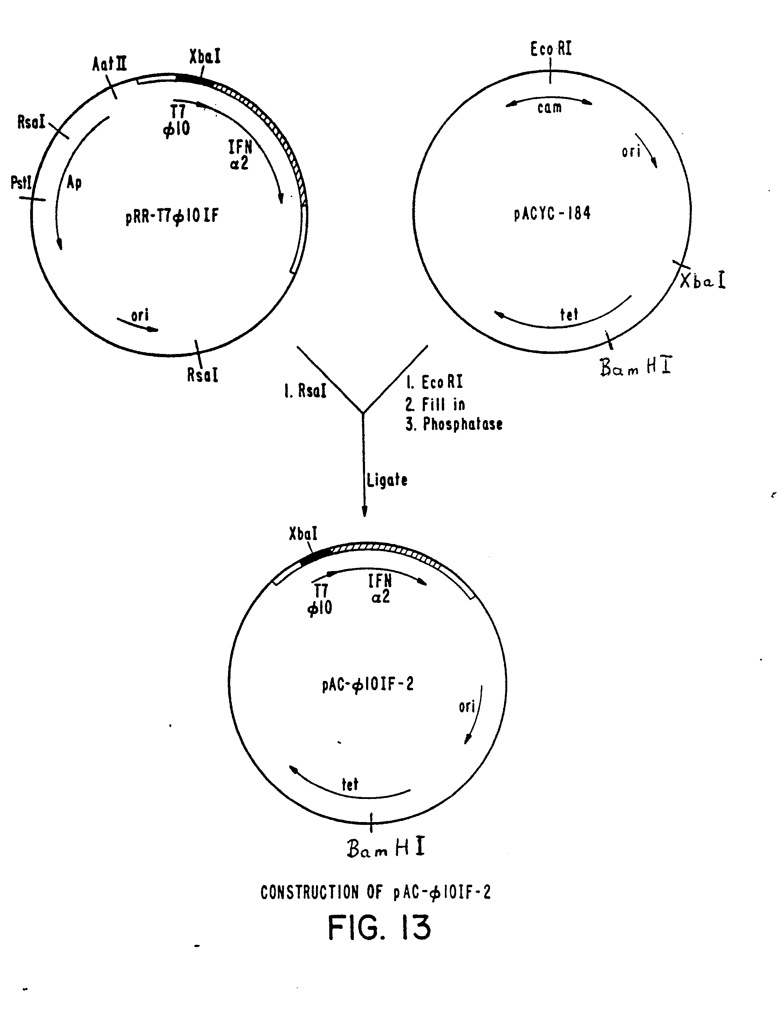

Since blunt-ended ligation was used to construct pAC-∅10IF-2, two orientations of the Rsa I fragment were possible. The screen indicated that some plasmids had one orientation, other plasmids the other orientation; so the orientation of pAC-∅10IF-2 was determined by performing restriction enzyme analysis with Xba I and Bam HI. The T7-∅10IF fragment was oriented in the clockwise direction according to the map shown in reference 7 (See Figure 13).

Improvement of the expression of the T7 RNA polymerase gene. After analysing the expression of the T7 polymerase (measured by the production of alpha-2 interferon), improvement in expression was made by treating the Eco RI T7 Gene 1 and 2 fragment of pAC-1T7G2-78 with the enzyme Bal 31 (Figure 14). The pBR322 DNA that separated the lac po element from the T7 DNA cloned from pT7-13 was removed in varying amounts by setting up the Bal 31 reaction so that approximately 400 base pairs would be removed from each end of the DNA fragment over a thirty minute time period. Samples were taken every five minutes and in this way a pool of fragments was created. After purification of the DNA, the fragments were digested with Bam HI.

The cloning vehicle was prepared by digesting p804-RI lac
with Pvu II and Bam HI. The entire pool of flush-ended
fragments of varying lengths were joined to the lac
promoter. The ligation pool was transformed into E. coli
D1210. The clones were screened by digesting with XmnI
and Eco RI. An XmnI restriction site exists 28 b.p.
after the ATG of the T7 RNA polymerase gene. Therefore,
the Eco RI/XmnI fragments created by the ligation of the
Bal 31 pool to the lac po were of various lengths owing
to the digestion by the Bal 31 enzyme. The clones were
classified by the length of this fragment which ranged
from 530 b.p. to 260 b.p. Clones were designated pRRB
followed by its number in the screen. One dozen clones
representing different Eco RI/XmnI fragment sizes were
transformed with pAC-Ø10IF (Figure 13) and tested for
alpha-2 interferon production upon induction by IPTG.
The results shown in Table 1 demonstrate the IPTG-
inducible production of alpha-2 interferon by the strain
harboring pRRB20. There was a significant increase of
interferon activity over the six hour test period in the
IPTG-induced culture while the uninduced culture showed
no significant increase in interferon activity.

The Eco RI/XmnI fragment representing the nucleotide
sequence from the Eco RI site in the lac po region to the
XmnI site located 28 b.p. after the ATG start codon of
the T7 polymerase gene was sequenced to determine the
number of nucleotides removed by the Bal 31 digestion.
It was determined that base pairs 2219 through 2196 of
the intervening pBR322 DNA were removed by the Bal 31
digestion. Therefore, the pBR322 DNA sequence that
separates the lac promoter from the T7 DNA in the pRRB20
construction runs from b.p. 2197 to b.p. 2067 of the
pBR322 sequence.

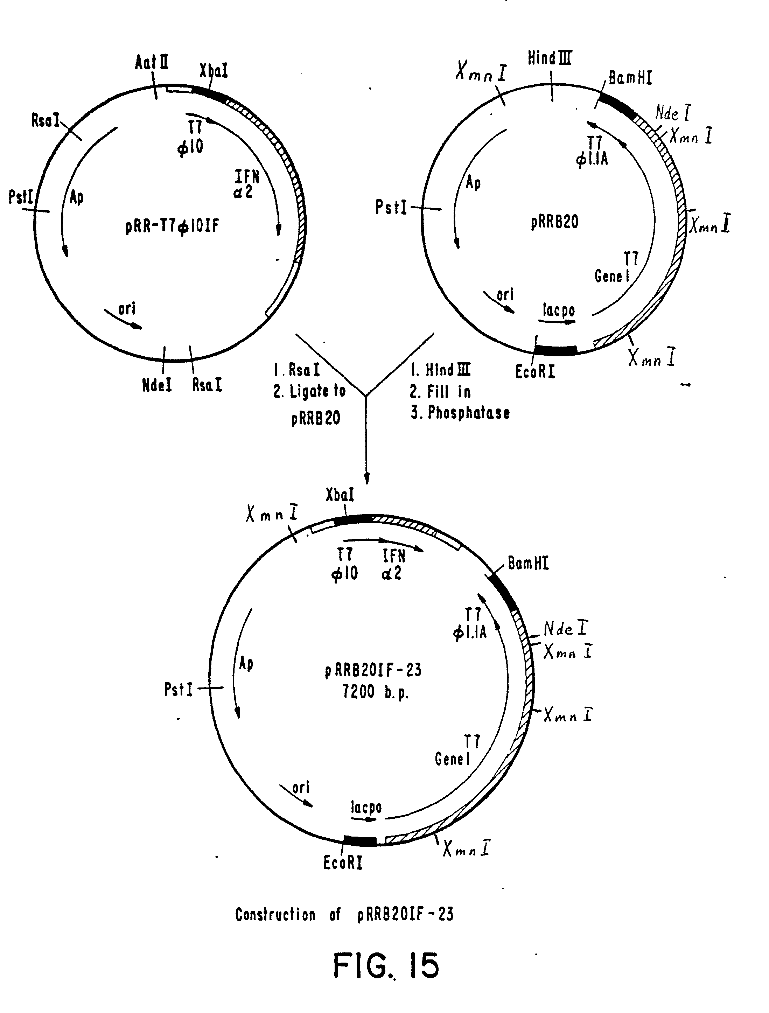

Cloning the fragment containing the T7 gene 10 promoter and alpha-2 interferon gene into pRRB20 (Figure 15). pRRB20 was digested with Hind III and the site was filled in with the Klenow enzyme. The DNA was precipitated with ethanol, resuspended in the appropriate buffer and treated with calf intestinal alkaline phosphatase. The DNA was isolated by agarose gel electrophoresis.

pRR-T7Ø10-IF was digested with Rsa I and the fragment containing the T7 gene 10 promoter and alpha-2 interferon gene was isolated by agarose gel electrophoresis. This fragment was ligated with the pRRB20 plasmid prepared above and the ligated DNA was transformed into E. coli D1210. Cloned plasmid DNA preparations were screened by digesting with Xmn I. After the initial screen, the construction was confirmed and the orientation was determined by digesting the clones with Xba I, a unique site in the T7 promoter, and with Nde I, a unique site in the T7 RNA polymerase gene. From this study, strain pRRB20IF-23 was selected as the prototype.

Testing the pRRB20IF-23 system for production of alpha-2 interferon. pRRB-20IF-23 was transformed into E. coli RB 791 and E. coli D1210. Both strains contain a chromosomal lac I$^q$ mutation. Induction experiments were run as described for the single plasmid system in the Methods section. The cells were grown at 30°C to cell densities of Klett 200 and then induced with IPTG at a final concentration of 5mM. At this time, the incubation temperature was raised to 37°C for the remainder of the experiment. Triplicate 1 ml samples were taken at two hour intervals and a final set of samples were made after continuous incubation overnight (about 20 hours post-induction). Table 2 shows the results of a typical experiment for each strain. The data demonstrate that there was a steady accumulation of alpha-2 interferon in the IPTG-induced cells. There was a 38-fold difference in the E. coli D1210 host strain and a 30-fold difference with E. coli RB 791 host strain.

A Contruction in which the T7Ø10-Directed Gene is in the same Reading Orientation as the lac Promoter T7 Gene 1

The versatility of the single plasmid system and utility of the various elements in different combinations and genetic orientations was demonstrated by the construction of the pRRB20C clones. Figure 16 demonstrates how pRRB2ØIF-23 and pKG-2, a hybrid cop$^{ts}$ cloning vehicle, were digested with the restriction enzyme Aha III and then with Eco RI. From pRRB20IF-23, the DNA fragment containing the lac po and T7 Gene 1 expression system was isolated by agarose gel electrophoresis; and from pKG-2 the DNA fragment encompassing the origin of replication and the tetracycline-resistance gene was isolated by agarose gel electrophoresis. After ligating the appropriate DNA fragments and transforming E. coli D1210 with the ligation mixture, clones were isolated and screened by digesting plasmid DNAs with the enzyme Bam HI. The close E. coli D1210/pRRB20C was further characterized by digesting with AhaIII, AvaI, EcoRI and KpnI (Figure 16).

A blunt-ended DNA fragment containing the T7Ø10-heterologous gene can be cloned into the single AhaIII restriction site (a blunt-end restriction cleavage) with the possibility of obtaining clones whose 5' to 3' reading orientations ran in either of the two directions. To demonstrate this, pRR20IF-23 was digested with AhaIII, and the fragment containing the T7 gene 10 promoter and alpha-2 interferon gene was isolated by agarose gel electrophoresis. pRRB20C was digested with AhaIII and then treated with calf intestinal alkaline phosphatase. The fragments containing the T7 promoter and the interferon gene were ligated with the pRRB20C vehicle and transformed into E. coli D1210. From the clones screened, several clones of each orientation, i.e., the same as or opposite to the 5' to 3' direction of the T7 Gene 1 transcript, were isolated. The orientation was confirmed by the size of the AvaI/AatII restriction fragment. Two clones, one of each orientation, were grown in flask cultures and induced with IPTG to demonstrate production of alpha-2 interferon by the T7-RNA polymerase. Clone pRRB20CIF-2 has its T7 Gene 10 and alpha-2-IFN gene sequence in the same 5' to 3' reading orientation as the lac po and T7 Gene 1 sequence. Clone pRRB20CIF-11 has the two sequences arranged in opposed orientations (Fig. 16). The induction procedure for these two cultures was the same as that used for the pRR20IF-23 clone except that the initial temperature was 30°C instead of 37°C. The temperature was shifted upon induction with IPTG to 42°C for 1 hr and then back to 37°C for further incubation, since the replication origin of the plasmids (see Fig. 16) was a temperature sensitive cop-mutant which is lethal to the cells at elevated temperatures (Haakkaart et al.), owing to the very high copy number of the plasmid. As can be seen by the data in Table 3 the plasmid system is inducible in either orientation. The fact that the promoters in pRR20CIF-2 were all situated in the same 5' to 3' orientation did not alter the inducibility or the stability of this single plasmid system.

Evidence for the Rifampicin-resistance of the T7 RNA
Polymerase-Directed Transcription:

The initiation of transcription by E. coli RNA polymerase
is highly sensitive to rifampicin. However, T7 RNA
polymerase, which is different in its structure and
characteristics, can initiate transcription from T7
promoters even in the presence of rifampicin. E. coli
D1210 strains containing the plasmid pRRB20IF-23 were
grown at $30°C$ to cell densities of Klett 200 and then
induced with IPTG at a final concentration of 2mM. At
this time the incubation temperature was raised to $37°C$
for the remainder of the experiment. Rifampicin
(100µg/ml) was added at 1.5, 5 hrs and 6 hrs after
induction with IPTG. Triplicate 1 ml samples were taken
at every 1.5 hr interval and assayed for IFN activity.

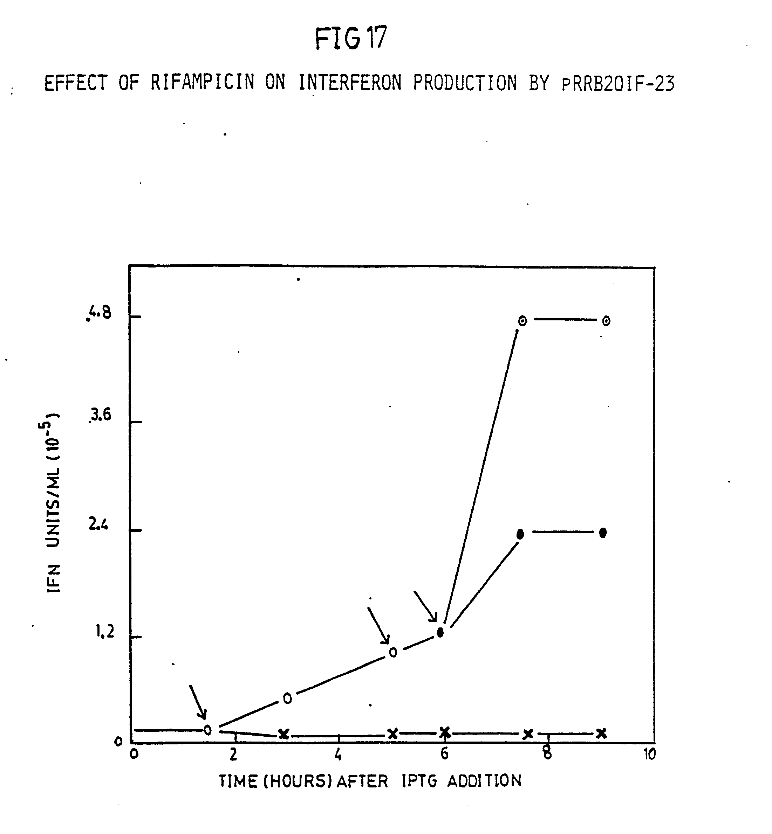

Fig. 17 shows the results of this experiment. When
rifampicin was added at the time of induction, there was
no production of interferon. However, when rifampicin
was added at 5 and 6 hours, there was continued high-
level synthesis of IFN. The difference in the absolute
levels of IFN production after the addition of rifampicin
also indicated that the concentration of T7 RNA
polymerase was steadily increasing for at least 6 hours
after induction. Under these circumstances, these cells
should be exclusively geared towards the production of
alpha-2 interferon, since rifampicin inhibits all
transcription dependent upon E. coli RNA polymerase.
Eventually the only mRNA being transcribed in these cells
is that directed by T7 RNA polymerase. Rifampicin
imitates the effect of T7 Gene 2 in a general sense.
Incorporation of a T7 Gene 2 into such a system would
essentially give the same results. Binding of E. coli
RNA polymerase by Gene 2 encoded protein will inactivate
it, bringing to a standstill all the host transcriptions. However, T7 RNA polymerase, being insensitive to Gene 2 protein, can continue transcribing until the host machinery is exhausted. This system therefore has the potential of yielding heterologous protein in a highly concentrated form.

## Construction of a T7 Ø10 Promoter Vector with Multiple Cloning Sites

To facilitate the cloning of other heterologous genes adjacent to the T7 Gene 10 promoter, the promoter was cloned into pMT11S so that the polylinker DNA of this plasmid was directly downstream of the T7 promoter. pMT11S (H. Huang and K. Moore, DNAX Res. Inst., personal communication), a general cloning vector, contains a polylinker sequence that has recognition  sequences for the following restriction enzymes: EcoRI, SmaI, BamHI, SalI, PstI, BglII, XbaI, HindIII.  pRRT7Ø10-18 (Fig. 17) was digested with NdeI.  This site was filled in by the Klenow reaction and the linear DNA was isolated by agarose gel electrophoresis.  The linear DNA thus purified was further digested with the enzyme PvuI, and the fragment containing the T7 Gene 10 promoter and the partial sequence of the ampicillin resistance gene was isolated by agarose gel electrophoresis.  pMT11S was digested with EcoRI, treated with the enzyme ExoVII and the linearized DNA was isolated by agarose gel electrophoresis.  This fragment was then restricted with PvuI.  The large fragment containing the polylinker sequence, the origin of replication and the 3' section of the ampicillin resistance gene was isolated by agarose gel electrophoresis.  These two fragments were then ligated together and transformed into E. coli 294.

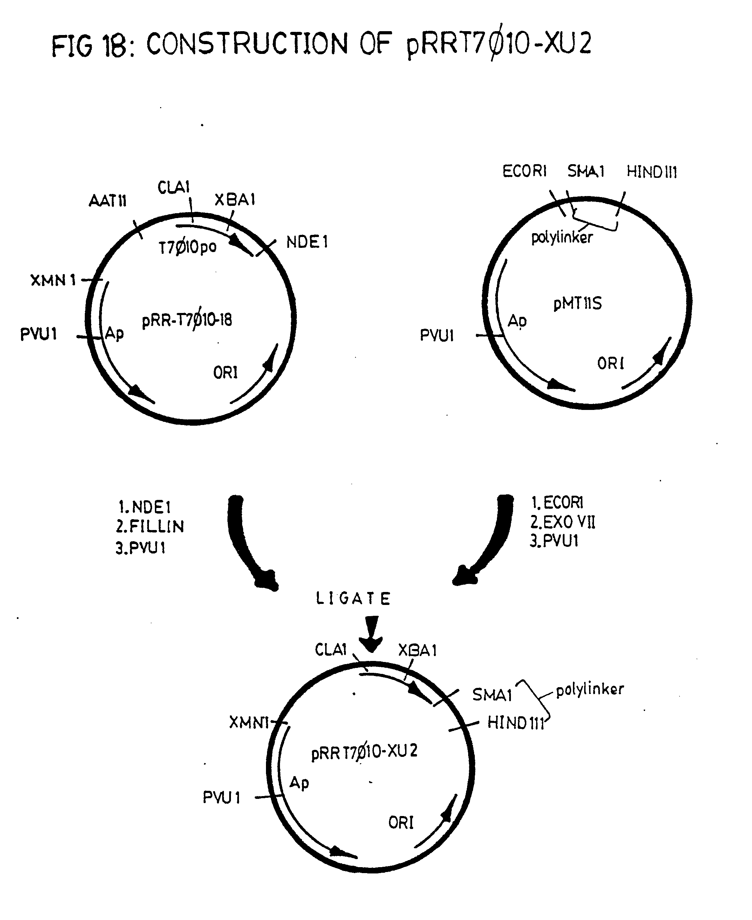

The selection used depended on the regeneration of the ampicillin resistance gene. Clones were screened by digesting plasmid DNA with XmnI, cleaving at a site unique to the pRRT7Ø10-18 DNA, and with HindIII, cleaving at a unique site in the pMT11S DNA. The plasmid pRRT7Ø10-Xu2 was isolated from this screen (Figure 18).

## Expression of other Heterologous Genes using T7 Single Plasmid System

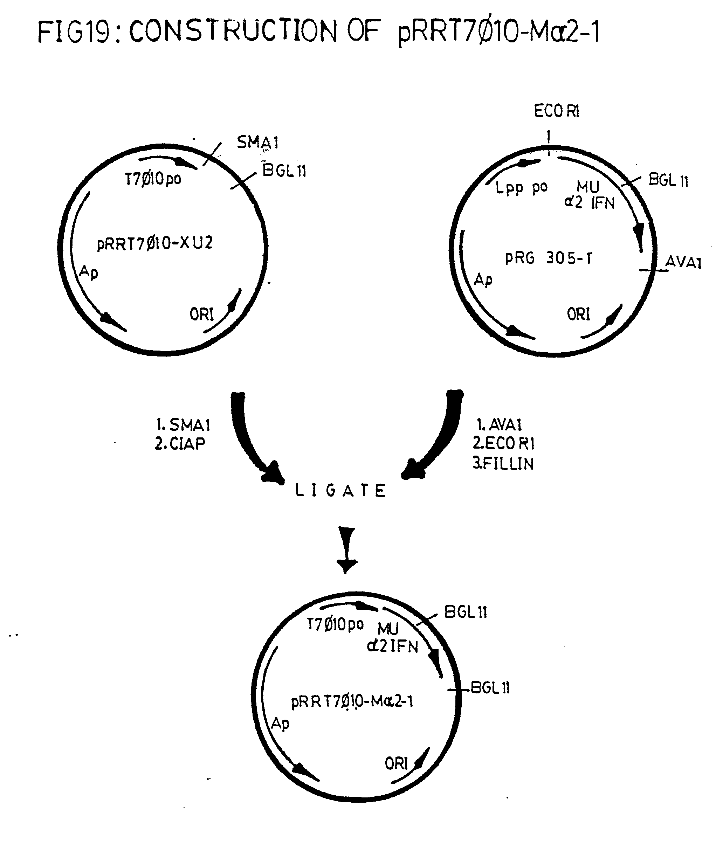

1. Cloning Mu alpha-2 Interferon gene under T7Ø10 Control The murine alpha-2 interferon gene can be obtained by standard methods, e.g. by digesting a plasmid containing it (Shaw et al., 1983). We obtained it by digesting a plasmid designated pTRG305-1 (provided by R. Greenberg, Schering Corp.) with the enzymes AvaI and EcoRI followed by a Klenow reaction. The DNA containing the murine alpha-2 interferon gene region was isolated by agarose gel electrophoresis as an approximately 1550 b.p. fragment. pRRT7Ø-Xu2 was digested with SmaI and then treated with calf intestinal alkaline phosphatase. The linear DNA was isolated by agarose gel electrophoresis. The DNA fragment containing murine alpha-2 interferon was ligated with this DNA and transformed into E. coli 294. Cloned plasmid DNAs were screened by digestion with the enzyme BglII. The size of the fragments observed by agarose gel electrophoresis established both the presence of the murine alpha-2 interferon gene and the orientation of the gene with respect to the T7 Gene 10 promoter. pRRT7Ø10-Mα2-1 was identified in this way (Fig. 19).

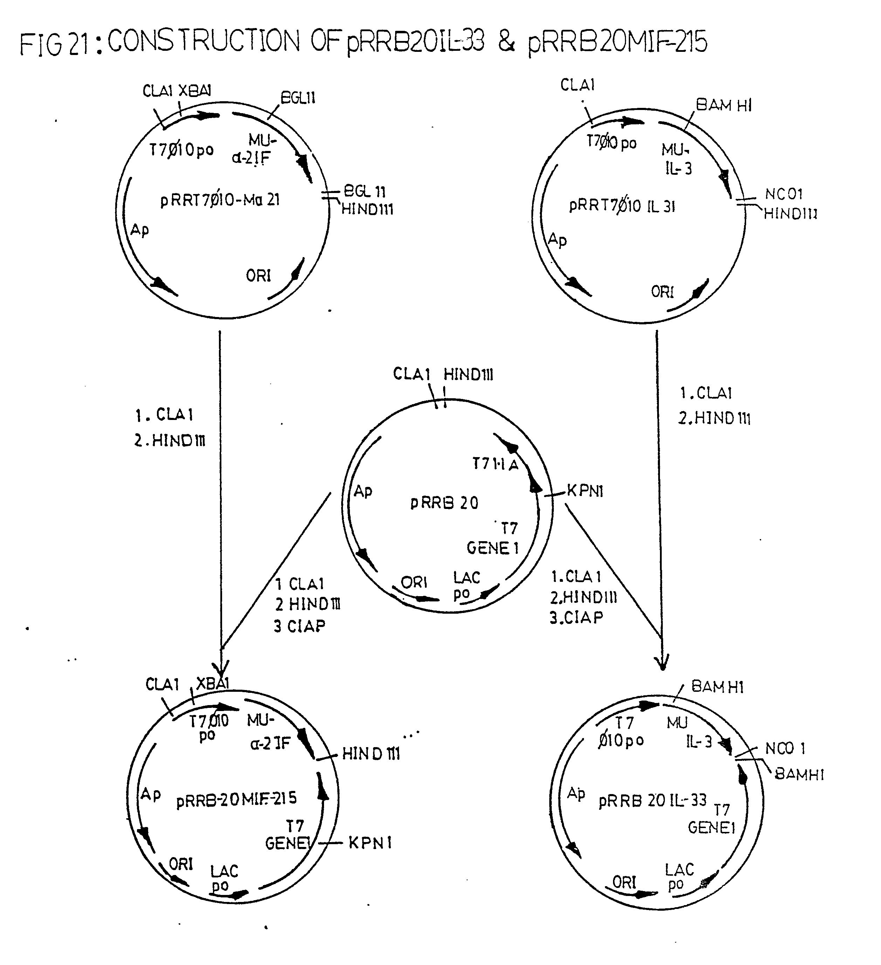

   pRRB20 and pRRTØ10-2-1 were digested with the enzymes ClaI and HindIII. pRRB20 was further treated with calf intestinal alkaline phosphatase. The appropriate fragments were then isolated by agarose gel
electrophoresis and the DNAs were ligated and
transformed into E. coli D1210.  Clones were screened
by digesting with ClaI and Hind III, and clones that
yielded a 1700 bp fragment were selected for further
experimentation. The clone chosen to test for
expression of the murine alpha-2 interferon gene was
designated pRRB-20MIF-215 (Fig. 21).  Upon induction
with IPTG, this clone produced approximately 60,000
units/ml of murine alpha-2 interferon.


2. Cloning of Murine IL-3 gene under T7Ø10 Control

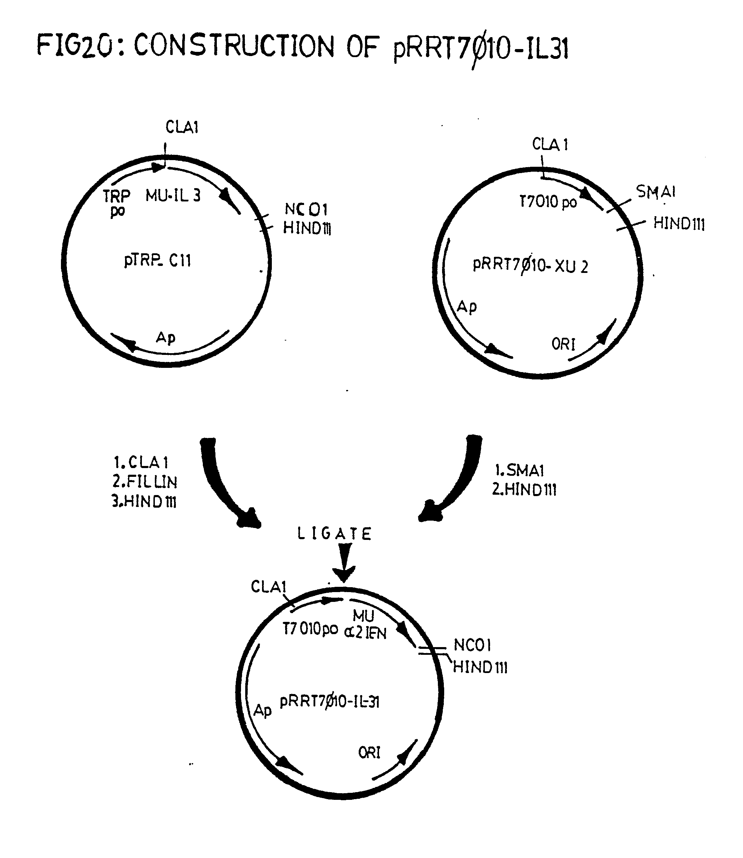

The murine interleukin-3 (IL-3) gene can be obtained
by standard methods (Yokota et al., 1984) and can then
be cloned into the pRRB20 following essentially the
same strategy as was used to clone the murine alpha-2
IFN gene.  We obtained a 470 b.p. fragment containing
the murine IL-3 gene by digesting a plasmid designated
pTRP-C11 (provided by G. Zurawski, DNAX Research
Institute, Palo Alto, California) with ClaI, filling
the site in with the Klenow enzyme and then digesting
further with HindIII restriction enzyme.  The fragment
containing the murine IL-3 gene was then cloned into
pRRØ10-XU2, which had been digested with the enzymes
SmaI and HindIII.  The clones were screened by
digesting with ClaI for which a digestion site is
present in the T7 DNA segment and with NcoI which is
located in the 3' prime end of the IL-3 DNA
fragment.  From this screen, pRRØ10-IL-31 was isolated
and characterized (Fig. 20).  The fragment containing
the T7 Gene 10 promoter and IL-3 DNA was then cloned
into pRRB20 in the same manner as described above for
the murine alpha-2 interferon gene (by using a
combined ClaI/HindIII digestion of each plasmid,
isolating the appropriate linear DNAs by agarose gel

electrophoresis and then ligating the T7Ø10-IL-3 gene fragment with the pRRB20 vehicle and transforming it into E. coli D1210). The clones isolated were screened by digesting plasmid DNA with the restriction enzymes NcoI and BamHI. A clone containing pRR20IL-33 was isolated from this screen (Fig. 21).

Expression was confirmed by lysis of induced cultures of this clone and analysis of the products on SDS-polyacrylamide gel; a band running at the molecular weight range of mu IL-3 was observed.

0178863

-41-

TABLE 1

Production of Alpha-2 Interferon by pRRB 20 by

inducing T7 RNA Polymerase Expression With IPTG

| Post-induction | Alpha-2 IFU/ml Klett Unit[*] | |
|---|---|---|
| Sample Time (Hrs.) | +IPTG** | -IPTG |
| 0 | 6.6 | 8.3 |
| 1 | 23.6 | 5.0 |
| 2 | 29.6 | 5.7 |
| 3 | 42.1 | 7.7 |
| 4 | 58.5 | 2.7 |
| 5 | 69.6 | 1.8 |
| 6 | 72.7 | 1.8 |

*Average of three samples.
**IPTG added to a final concentration of 5mM.

-42-

## TABLE 2

### Production of alpha-2 interferon in E. coli strains harboring pRRB20IF-23 upon induction with IPTG

| Strain | Post-induction Sample Time (hrs.) | IFU/ml Klett Unit[*] | |
| --- | --- | --- | --- |
| | | (+) IPTG (5mM) | (-) IPTG |
| E.c. D1210 | 0 | 40.0 | 40.0 |
| | 2 | 32.0 | 22.0 |
| | 4 | 129.0 | 16.0 |
| | 6 | 222.2 | 13.0 |
| | 20 | 578.3 | 15.4 |
| E.c. RB791 | 0 | 25.0 | 25.0 |
| | 2 | 80.0 | 23.8 |
| | 4 | 105.8 | 18.9 |
| | 6 | 370.4 | 17.3 |
| | 20 | 213.3 | 6.8 |

*Average of three samples.

Table 3

Production of alpha-2 Interferon in E. coli D1210

harboring pRRB20CIF-2 or pRRB20CIF-11 upon Induction

with IPTG

| Post-induction Sample Time (hrs) | | IFU/ml Klett Unit | |
|---|---|---|---|
| | | +IPTG(5mM) | IPTG |
| | 0 | 5.6 | 5.6 |
| | 1 | 14.2 | 10.7 |
| pRRB20CIF-2 | 2 | 35.7 | 17.2 |
| | 4 | 288.6 | 41.0 |
| | 6 | 297.6 | 83.3 |
| | 20 | 240.0 | 4.2 |
| | 0 | 3.4 | 3.4 |
| | 1 | 34.8 | 28.6 |
| pRRB20CIF-11 | 2 | 57.1 | 52.6 |
| | 4 | 147.4 | 90.0 |
| | 6 | 448.0 | 123.0 |
| | 20 | 290.9 | 78.6 |

The following Bibliography refers to references given in the above Materials and Methods Section.

1.      Backman, K., M. Ptashne, and W. Gilbert. 1976. Construction of plasmids carrying the Cl gene of bacteriophage lambda. Proc. Nat. Acad. Sci., 73 4174-4178.

2.      Bolivar, F. and K. Backman 1979. Plasmids of Escherichia coli as Cloning Vectors. In Wu, R. Methods of Enzymology 68: 245-267.

3.      Brent, R. and M. Ptashne, 1981. Mechanism of action of the lex A gene product. Proc. Nat. Acad. Sci., 78: No. 7 pp 4204-208.

4.      Chang, A.C.Y. and S.N. Cohen. 1978. Construction and characterization of amplifiable multicopy DNA cloning vehicles derived from P15A cryptic miniplasmids. J. Bacteriol. 134: 1141

5.      Dagert, M. and S. D. Ehrlich. 1979. Prolonged Incubation in Calcium Chloride Improves the Competence of Escherichia coli cells. Gene 6: 23-28.

6.      Dunn, J.J. and F. Studier, 1983. Complete Nucleotide Sequence of Bacteriophage T7 and the Locations of T7 Genetic Elements. J. Mol. Biol. 166: 477-535.

7.      Familletti, Phillip C., Sara Rubenstein, and Sidney Pestka. 198. Methods in Enzymology, 78: 387-394. Academic Press Inc.

8.      Maniatis, T., E.F. Fritsch, and J. Sambrook.
        1982.  Molecular Cloning: A Laboratory Manual.
        Cold Spring Harbor Laboratory.


9.      Messing, J. and J. Vieira 1982.  A new pair of
        M13 vectors for selecting either DNA strand of
        double-digest restriction fragments. Gene 19,
        269-276.


10.     Sadler, J., R.M. Tecklenburg, J.L. Betz. 1980.
        Plasmids containing many tandem copies of a
        synthetic lactose operator.  Gene 8: 279-300.


11.     Sanger, F., S. Nicklen, and A. R. Coulson
        1977.  DNA sequencing with chain-terminating
        inhibitors.  Proc. Natl. Acad. Sci. USA. vol 74
        No. 12 pp. 5463-5467.


12.     Smith, H.O. 1980. "Recovery of DNA from gels,"
        Methods in Enzymology, Vol. 65, Part I, edited
        by Grossmand, L. and K. Moldave. Academic Press,
        N.Y. 371-380.


13.     Stahl, S. and K. Zinn. 1981.  Nucleotide
        Sequence of the Cloned Gene for Bacteriophage T7
        RNA polymerase. J. Mol. Biol. 24: 481-485.


14.     Sutcliffe, J. G. 1978. pBR322 restriction map
        marked from the DNA sequence: Accurate DNA size
        markers up to 4361 nucleotide pairs long.
        Nucleic Acids Res. 5: 2721.


15.     Hakkaart, M.J.J., E. Veltkamp and H.J.J.
        Nykamp:  Protein H Encoded by Plasmid CloDF-13 Involved in Lysis of the Bacterial Host II. Functions and Regulation of Synthesis of the Gene H Product. Mol. Gen. Genet. 183 (1981) 326-332.

16.    Laemmli, U.U. 1970.  Cleavage of Structural Proteins during the Assembly of the Head of Bacteriophage $T_4$. Nature.  227: 680-685.

17.    Shaw, Gray D., W. Boll, H. Taira, N. Manter, P. Lengyel and C. Weissmann.  1983.  Structure and Expression of Cloned Murine IFN-α Genes. Nucleic Acids Research Vol 11: 555-573.

18.    Yokota, T., F. Lee, D. Rennick, C. Hall, N. Arai, T. Mossmann, G. Nable, H. Cantor and K. Arai.  1984.  Isolation and Characterization of a Mouse cDNA Clone that Expresses Mast-cell Growth-factor Activity in Monkey Cells.  PNAS 81: 1070-1074.

CLAIMS:

1.        A method of producing a heterologous polypeptide in an appropriate host, comprising culturing, under suitable conditions that favor the expression of the heterologous polypeptide, host cells which contain a recombinant plasmid vector that comprises a compatible inducible promoter region which is followed by and is in the same transcriptional direction as the bacteriophage T7 RNA polymerase gene; and further which contain a bacteriophage T7 promoter region which is followed by and is in the same transcriptional direction as a structural DNA sequence which specifies said heterologous polypeptide; wherein said T7 promoter region and structural DNA sequence are on the above-mentioned recombinant plasmid vector or on a second recombinant plasmid vector.

2.        A method of producing a heterologous polypeptide in an appropriate host, comprising culturing, under suitable conditions that favor the expression of the heterologous polypeptide, host cells which contain one or more copies each of:

> a) a first recombinant plasmid vector that comprises a compatible inducible promoter region which is followed by and is in the same transcriptional direction as the bacteriophage T7 RNA polymerase gene; and
> b) a second recombinant plasmid vector that comprises a bacteriophage T7 promoter region which is followed by and is in the same transcriptional direction as a structural DNA sequence which specifies said heterologous polypeptide.

3.     A method for producing a heterologous polypeptide in an appropriate host, comprising culturing under suitable conditions that favor the expression of the heterologous polypeptide, host cells which contain one or more copies of a recombinant plasmid vector, said vector comprising:

   a) a compatible inducible promoter region which is followed by and is in the same transcriptional direction as the bacteriophage T7 RNA polymerase gene; and

   b) a bacteriophage T7 promoter region which is followed by and is in the same transcriptional direction as a structural DNA sequence coding for said heterologous polypeptide.

4.     A method as claimed in claim 3 wherein said bacteriophage T7 promoter region is oriented in the recombinant plasmid vector in the direction opposite to the direction required to transcribe the bacteriophage T7 RNA polymerase gene.

5.     A method as claimed in claims 1 to 4 wherein the host is a bacterium, e.g. E. coli.

6.     A DNA fragment which comprises a bacteriophage T7 promoter region which is coupled to and is in the same transcriptional direction as a structural DNA sequence which specifies a desired polypeptide, especially when contained in a recombinant plasmid vector which is suitable for transformation into a host bacterial cell.

7.      A DNA fragment which comprises:
        a) an inducible promoter region which is
           coupled to and is in the same
           transcriptional direction as the
           bacteriophage T7 RNA polymerase gene; and
        b) a bacteriophage T7 promoter region which is
           followed by and is in the same
           transcriptional direction as a structural
           DNA sequence which specifies a desired
           polypeptide;

especially when contained in a recombinant plasmid vector
which is suitable for transformation into a bacterial
host cell.

8.      A DNA fragment as claimed in claim 7 wherein
said bacteriophage T7 promoter region is oriented in the
recombinant plasmid vector in the direction opposite to
that required to transcribe the bacteriophage T7 RNA
polymerase gene.

9.      A DNA fragment as claimed in claim 7 wherein
said bacteriophage T7 promoter region is oriented in the
recombinant plasmid vector in the same direction as that
required to transcribe the bacteriophage T7 RNA
polymerase gene.

10.     A transformed host cell, especially a
bacterium, which comprises at least one copy of a
recombinant plasmid vector, said vector comprising a
bacteriophage T7 promoter region which is coupled to and
is in the same transcriptional direction as a structural
DNA sequence which specifies a desired polypeptide, and
which in addition preferably comprises at least one copy
of a second recombinant plasmid vector, said vector comprising a compatible inducible promoter region which is coupled to and is in the same transcriptional direction as the bacteriophage T7 RNA polymerase gene.

11.     A transformed host, especially a bacterium, which comprises at least one copy of a recombinant plasmid vector, said vector comprising:

   a) a compatible inducible promoter region which is followed by and is in the same transcriptional direction as the bacteriophage T7 RNA polymerase gene; and

   b) a bacteriophage T7 promoter region which is followed by and is in the same transcriptional direction as a structural DNA sequence which specifies a desired polypeptide.

12.     A transformed host as claimed in claim 11 wherein said bacteriophage T7 promoter region present in the recombinant plasmid vector is oriented in the direction opposite to the direction required to transcribe the bacteriophage T7 RNA polymerase gene which is also present in said vector.

13.     A recombinant plasmid vector comprising a bacteriophage T7 promoter region and, directly downstream thereof, at least one restriction site for insertion of a structural DNA sequence specifying a desired polypeptide.

DIAGRAM OF pRRl9   FIG. I

DIAGRAM OF pRR-IT7-39   FIG. 2

# FIG. 3

## CONSTRUCTION OF p804-RI lac

CONSTRUCTION OF pRR-1T7L-3

# FIG. 4

0178863

4/20

CLONING OF T7 Gene 2

FIG. 5

5/20

0178863

DIAGRAM OF pRR-2T7-35

FIG. 6

DERIVATION OF pRR-1B-2T7 FROM pRR-2T7-35

FIG. 7

Insertion of BamHI linkers into NdeI
site of pRR-IB-2T7

FIG. 8

Construction of pRR-1T7G2 lac 7

# FIG. 9

DIAGRAM OF pAC·1T7G2-78

FIG. 10

CLONING OF THE T7 GENE 10 PROMOTER

FIG. 11

Cloning the T7 Gene 10 promoter adjacent to the IFN-Alpha 2 gene

FIG. 12

CONSTRUCTION OF pAC-φ10IF-2

FIG. 13

Bal 31 digestion of the region between the _lac_ promoter and T7 Gene I

FIG. 14

Construction of pRRB20IF-23

FIG. 15

FIG 16: CONSTRUCTION OF pRR20CIF-2

## FIG 17

EFFECT OF RIFAMPICIN ON INTERFERON PRODUCTION BY pRRB20IF-23

# FIG 18: CONSTRUCTION OF pRRT7Ø10-XU2

# FIG19: CONSTRUCTION OF pRRT7Ø10-Mα2-1

# FIG20: CONSTRUCTION OF pRRT7010-IL31

20/20          0178863

# FIG 21 : CONSTRUCTION OF pRRB20IL-33 & pRRB20MIF-215

**European Patent Office**

# EUROPEAN SEARCH REPORT

| | DOCUMENTS CONSIDERED TO BE RELEVANT | | EP 85307299.9 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | PROCEEDINGS OF THE NATIONAL ACADEMY OF SCIENES OF THE UNITED STATES OF AMERICA, vol. 81, no. 7, April 1984 (Baltimore, USA)<br><br>P. DAVANLOO et al. "Cloning and expression of the gene for bacteriophage T7 RNA polymerase" pages 2035-2039<br><br>* Totality * | 1-3,5, 6,10, 11 | C 12 N 15/00<br>C 12 P 21/00<br>C 07 H 21/04<br>C 12 N  5/00<br>C 12 N  1/20<br>//C 12 R  1:19 |
| D,A | JOURNAL OF MOLECULAR BIOLOGY, vol. 153, November 25 - December 25, 1981 (London, New York, San Francisco)<br><br>W.T. MC ALLISTER et al. "Utilization of Bacteriophage T7 Late Promoters in Recombinant Plasmids during Infection" pages 527-544<br><br>* Pages 527, 528 * | 1-3,6, 10,11 | |
| | | | **TECHNICAL FIELDS SEARCHED (Int. Cl.4)**<br><br>C 12 N<br>C 12 P<br>C 07 H |

The present search report has been drawn up for all claims

| Place of search | Date of completion of the search | Examiner |
|---|---|---|
| VIENNA | 14-01-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82